# EUROPEAN PATENT APPLICATION

(11) **EP 3 925 626 A1**
(43) Date of publication of application: **22.12.2021**
(21) Application number: 21020127.3
(22) Date of filing: 05.03.2021
(51) Int. Cl.: A61K 47/54, C07K 7/06

(54) **LIPOPEPTIDE CONJUGATES AND THEIR SALTS, METHOD OF MANUFACTURE OF LIPOPEPTIDE CONJUGATES AND THEIR SALTS, PHARMACEUTICAL COMPOSITION COMPRISING A LIPOPEPTIDE CONJUGATE AND ITS SALTS FOR THE TREATMENT OF CHRONIC WOUNDS**

(30) Priority: 06.03.2020 PL 43316920
(71) Applicant: Gdanski Uniwersytet Medyczny, 80-210 Gdansk (PL); Politechnika Gdanska, 80-233 Gdansk (PL)
(72) Inventor: Lahutta, Monika, 80-807 Gdansk (PL); Pikula, Michal, 80-170 Gdansk (PL); Dzierzbicka, Krystyna, 80-041 Gdansk (PL); Schumacher, Adriana, 76-024 Konikowo (PL); Sass, Piotr, 81-577 Gdynia (PL); Sosnowski, Pawel, 19-200 Ruda (PL); Sachadyn, Pawel, 81-646 Gdynia (PL); Wardowska, Anna, 80-297 Banino (PL); Nowicka, Ewa, 80-177 Gdansk (PL); Baczynski-Keller, Jakub, 81-107 Gdynia (PL)
(74) Representative: Godlewski, Piotr

(57) **Abstract**

**Object of the invention**

The object of the invention is a method of obtaining lipopeptide conjugates especially rich in L-hydroxyproline residues described by the general formula *N^{α}-acyl-(AK₅-AK₄*-*AK₃-AK₂-AK₁)ₙ-NH₂ (I)* or their pharmaceutically acceptable salts, wherein: AK₁,AK₂,AK₃,AK₄,AK₅ denote amino acid residues or their lack; AK₁ is a lysine residue of L or D configuration; AK₂ is an amino acid residue selected from the group consisting of non-polar neutral amino acids: Phe, Ala, Gly, Ile, Leu, Met, Pro, Val, Trp or its lack; AK₃ is an amino acid residue selected from Hyp, Phe, Ala, Gly, Ile, Leu, Met, Pro, Val, Trp; AK₄ is an amino acid residue selected from Pro, Hyp or its lack; AK₅ is the Gly residue; *N*^{α}-acyl group has the formula R₁-C(=O), wherein R₁ is a saturated carbon chain of length from C₇ to C₁₃; n denotes a number of repeat units from 1 to 2 of a general formula: AK₅-AK₄-AK₃-AK₂-AK₁.

In addition, the invention concerns the use of a pharmaceutical composition containing at least one lipopeptide conjugate or its pharmaceutically acceptable salt or a preparation for the stimulation of proliferation and migration of fibroblasts and keratinocytes, containing the pharmaceutical composition as a basic component for the manufacture of a wound dressing in the form of an aqueous solution, a suspension, an emulsion, an aerosol, a cream, an ointment, a three-dimensional scaffold, a hydrogel in a tube, for the use in the treatment of deep full-thickness wounds, i.e. burn wounds (second and third degree), venous leg ulcers, bedsores or metabolic disorders i.e diabetic foot, being a complication of existing cardiovascular disease or diabetes mellitus.

## Description

### Object of the invention

The object of the invention is a method of obtaining lipopeptide conjugates especially rich in L-hydroxyproline residues described by the general formula *N^{α}-acyl-(AK₅-AK₄-AK₃-AK₂-AK₁)ₙ-NH₂ (I)* or its pharmaceutically acceptable salts, wherein: AK₁,AK₂,AK₃,AK₄,AK₅ denote amino acid residues or their lack; AK₁ is a lysine residue of L or D configuration; AK₂ is an amino acid residue selected from the group consisting of non-polar neutral amino acids: Phe, Ala, Gly, Ile, Leu, Met, Pro, Val, Trp or its lack; AK₃ is an amino acid residue selected from Hyp, Phe, Ala, Gly, Ile, Leu, Met, Pro, Val, Trp; AK₄ is an amino acid residue selected from Pro, Hyp or its lack; AK₅ indicates a glycine residue; *N*^{α}-acyl group has the formula R₁-C(=O), where R₁ is a saturated carbon chain of length from C₇ to C₁₃; n denotes a number of repeat units from 1 to 2 of a general formula: AK₅-AK₄-AK₃-AK₂-AK₁.

In addition, the invention concerns the use of a pharmaceutical composition containing at least one lipopeptide conjugate or its pharmaceutically acceptable salt or a preparation for the stimulation of proliferation and migration of fibroblasts and keratinocytes, containing the pharmaceutical composition as a basic component for the manufacture of a wound dressing in the form of an aqueous solution, a suspension, an emulsion, an aerosol, a cream, an ointment, a three-dimensional scaffold, a hydrogel in a tube, for the use in the treatment of deep full-thickness wounds , i.e. burn wounds (second and third degree), venous leg ulcers, bedsores or metabolic disorders i.e diabetic foot, being a complication of existing cardiovascular disease or diabetes mellitus.

### State of technology

### Synthesis of peptide chain containing Hyp residue

The synthesis of hydroxyproline-rich peptide chain and their derivatives is currently an important direction of work on the stability of the triple structure of the tightly packed α-helix of collagen. The collagen chain, which is a component of the ECM (extracellular matrix), is formed by regularly repeating amino acid triads (Gly-X-Y) containing X proline residue and Y hydroxyproline (Hyp) or hydroxylysine (Hyl) residue - products of posttranslational modification (hydroxylation), affecting its increased stability. In light of the invention, it has become crucial in the design and development of an effective and rapid method to obtain novel lipopeptide conjugates with high eficiency and high degree of purity. The preparation of Hyp residue-rich conjugates and derivatives is carried out both in homogeneous solution and using solid-phase synthesis. In both cases the coupling reactions with hydroxyamino acid derivatives, i.e. the applied coupling reagents, protecting groups or reaction conditions are important for the final product.

The protection of the functional group in the hydroxyamino acid side chain in peptide synthesis limits the formation of undesired products resulting from *O*-acylation during peptide bond formation. The acylation of alcohols is a relatively easy reaction involving derivatives with a free carboxylic group and occurs with a large amount of available carboxylic acids. The rate of acylation step depends on whether it is a primary or a secondary hydroxyl functional group. The primary hydroxyl group of the serine residue or the phenolic group of the tyrosine residue is acylated quite easily, in particular in the presence of proton-withdrawing reagents, standard antiracemic additives used in the synthesis, for example HOBt, and coupling reagents, in particular carbodiimide derivatives. In contrast, the secondary hydroxyl group, for example of the threonine residue, may be unreactive during the coupling reaction, and so in some cases was not protected during the solution-phase synthesis. In Fmoc or Boc-based solid-phase synthesis, the practice is to use protection in the hydroxyamino acid side chain, where the activated carboxyl component is used in an excess.

Pfp or Dhbt esters of the amino acid residue in the presence of HOBt are beneficial to obtain a product rich in hydroxyamino acid residues on the polymeric support. Active esters are particularly recommended, being an alternative to other coupling reagents, i.e. replacing carbodiimide derivatives. Their use in the solid-phase synthesis of peptides rich in hydroxyamino acid residues avoids direct substitution of hydroxyl group.

This method of peptide bond formation has become useful in many laboratories because of the convenience of performing it in an automated synthesis process. However, the condensation reactions rate using Pfp esters of threonine residues were slower than those using other amino acid residues, required repetition and led to the contamination of the final product by fragments with additionally attached threonine residues.

Selecting the optimal coupling reagent and reaction conditions are important in efficient bond formation and elimination of side chains products. The use of the most popular *in situ* coupling reagents i.e. carbodiimide derivatives may lead to the formation of *O*-acylation products of hydroxyamino acid residues during the synthesis.

The formation of benzotriazole anhydrides or esters during the activation step with BOP may, under appropriate conditions, eliminates the adverse side reactions with threonine residue. The previous works with the use of Hyp derivatives in the synthesis are limited. The synthesis of linear peptides containing the Hyp residue dates back to 1944, when the first sequences, i.e. Gly-4-Hyp-L-Pro-Gly, 4-Hyp-L-Pro-Gly, derivatives involving 4-Hyp, di- or tripeptides were obtained. At this time, the efficient synthesis of angiotensin II analogues (Ile5-Hyp7-Angiotensin II) was described. Although condensation reactions were performed using the limited azide method, the difficulties in peptide bond formation or product isolation were encountered. In the synthesis of octapeptide, the Cbc(Cbz)-Hyp-Phe-ONBzl derivative was obtained in 77% yield by performing the *N*-protected Hyp residue to mixed anhydride with ethyl chloroacetate in the presence of tri-*n*-butylamine with aminocomponent acylation. The HBr salt of dipeptide was further reacted with the acylating reagent (an appropriate azide) to obtain the product for further condensation reaction. However, acylation was carried out using dipeptide azide (Cbz-Ile-His-N₃), to receive the required product in higher effieciency. The resulting HBr salts of methyl or p-nitrobenzyl esters of tripeptide (His-Hyp-Phe) were difficult to isolate due to significant contamination of the product.

The condensation reactions involving the Hyp residue as an amino component in the linear peptides have been carried out in particular with rarely used acyl reagents, i.e. acyl chloride or azide, but also with active esters, i.e. *N*-hydrocisucinimide esters. Cbz-Gly-Hyp-OH derivative was obtained using protected *N*-hydroxysuccinimide ester (Cbz-Gly-OSu) with 50÷60% yield. The mixed anhydride methods for peptide bond formation did not present significant differences. However, condensation reactions using active esters of *N*-protected Hyp (Cbz-Gly-Hyp-OSu) with various amino acid derivatives, for example: Hyp-OH, Hyp-OMe^{∗}HCl, Leu-OH were not efficent.

In most cases, no desired coupling products were obtained or the yields of the process were unsatisfactory. Amino acid has been coupled to the *N*-protected carboxylic Hyp component (Cbz-Gly-Hyp-Hyp-OBzl) by the mixed anhydride method or the carbodiimide method. Further attempts to obtain various structures with Hyp residues were a result of the growing interest in biologically active peptide structures. Cbz-Pro-4-Hyp-OH was efficiently obtained using the active ester i.e. Cbz-Pro-OSu or with the acid chloride Cbz-Pro-Cl or by saponification of Cbz-Pro-4-Hyp-OMe. However, analogously, no Cbz-Pro-Hyp-Gly was obtained using the active ester of Cbz-Pro-OSu, despite the modification of the reaction conditions, i.e. temperature or concentration of substrates.. The product was also not obtained using the carbodiimide method or using Cbz-Pro-4-Hyp-OH and HBr^{∗}Gly-ONp. In results, the desire product was obtained using dipeptide derivative (Cbz-4-Hyp-Gly) followed by condensation reaction with the L-proline p-nitrophenyl ester hydrobromide (HBr^{∗}Pro-ONp) using a carbodiimide derivative. Alternatively, this product was also obtained as a result of condensation reaction of Cbz-Pro-4-Hyp with HCl^{∗}Gly-OEt, followed by appropriate saponification.

Combined with the development synthetic and analytical methods, the need to develop the protecting groups for side chain of hydroxyproline was pointed out. Protecting groups for the side chain (hydroxyl group) of the hydroxyproline were investigated, of which the benzyl group (Bzl) was selected as the most suitable. The Boc-Hyp(Bzl)-OH derivative was used to obtain oligopeptides, i.e. Pro-Hyp-Gly, Hyp-Pro-Gly. The condensation reaction between Boc-Hyp(Bzl)-OH and HCl^{∗}Pro-OBzl was carried out using DCU in the presence of TEA. After removal of the benzyl ester, the dipeptide derivative, Boc-Hyp(Bzl)-Pro-OH, was conjugated with Gly-OEt. followed by hydrolysis to obtain the product with a yield of 75%.

In the SPPS according to the Fmoc strategy, various Hyp derivatives have been used. Side chain protecting group of hydroxyproline was orthogonally removed after synthesis, or as well selectively modified to form substituted proline derivatives.

Among the most common derivatives commercially available, Fmoc-4R-Hyp-OH or Fmoc-4R-Hyp(OtBu)-OH were used. Tert-butyl is a commonly used protecting group of hydroxyl group in Hyp residue, but not easily removed selectively in SPPS. It is stable under most mild alkaline conditions, while trifluoroacetic acid (95%) is preferred for its deprotection. However, the use of 9-fluorenylmethoxycarbonyl hydroxyproline derivative (Fmoc-Hyp-OH) with a free hydroxyl group to build the peptide loaded Rink Amide resin was described in the literature. However, then the trityl was easily introduced into the functional group of Hyp acting with trityl chloride (optionally imidazole) in the presence of DIPEA in DMF.

The protected dipeptide Fmoc-Hyp(OTrt)Asn(Trt)-NH₂ was then used to obtain the desired sequence Thr(OtBu)Tyr(Otbu)Hyp(OTrt)Asn(Trt)-NH₂ using a HBTU in the presence of DIPEA in DMF. The trityl group is selectively removed using DCM-TES-TFA (93:5:2, *v*/*v*/*v*), while the cleavage of the peptide from the resin occurs as standard solutions of TFA.

The strategies have to be developed for the synthesis of peptide chain of at least one but also several of the hydroxyl amino acid residues. The orthogonal protection of the hydroxyl group as tert-butyldimethylsilyl (TBS) ether was accomplished by treating with TBSCl. Deprotection of sillyl ether was achieved with TBAF followed by cleavage of the peptide chain from the Rink Amide resin. Alloc carbonate was also used protecting group to block hydroxyl functionalities. The Alloc group was introduced using allyl chloromethane, in the presence of DMAP and triethylamine, in toluene. The strategy was not employed in automated synthesis. It required an elevated temperature (50°C) and optimized deprotection conditions. However, it remained stable during deprotection steps and cleavage of the resin with a mixture of TFA.

In the case of Hyp residue, side chain *O*-acylation/alkylation takes place during amino acid coupling. Hyp-containing peptides were easily acylated in SPPS using a carbodiimide derivative (DIPCI) in the presence of a catalyst (DMAP), yielding the main *O-*acylation products with good efficiency after final deprotection with a standard TFA mixture. The acylation of Hyp side chain was achieved with biotin to enhance cognition, but also occurred with polar amino acids or peptides.

An important part of peptide synthesis is to receive repeating peptidyl units (fragment condensation), known as polycondensation. The first, successfully completed solid-phase synthesis providing the polycondensation products (Gly-X-Y)n was described by Sakakibara et al. The polycondensation process of various peptide units (monomers) with Pro, Hyp residues is mostly performed with the use of Boc strategy on Merffield resins or, MBHA (benzyhydrylamine hydrochloride bound to the polymer), resulting in the product release after HF treatment.

In the polycondensation (fragment condensation) approach, Fmoc chemistry was also used to build protected peptide fragments loaded polystyrene resin. A 28-amino acid polypeptide chain was synthesized by fragment condensation on Fmoc-Gly-2-chlorotrityl resin. Coupling reactions required in each case a 4-fold excess (relative to resin loading/substitution) of monomer to generate HATU active ester in the presence of NMM. The final product cleaved from the resin using a standard TFA mixture was free of impurities, however, the low yield (15%) of the product purified by RP-HPLC (scale: 50 µmol) prompts to an intensive search for other synthetic strategies. The synthesis of peptidyl fragments with a purity required to polycondensation reactions are performed in a homogeneous system. They require purification after each coupling step using standard techniques applied in organic chemistry, i.e. crystallisation, chromatographic techniques. Described in the literature, a classical (solution-phase) synthesis deliver high yields of purified short fragments of required peptides found in the collagen structure, that were directly used in polycondensation reactions. The synthetic methodology relied on the coupling reactions of amino acid residues using isobutyl chloroacetate to form an anhydride intermediate, or using the aforementioned pentafluorophenyl esters (Pfp). The resulting products were easy to separate. However, the successive steps of the synthesis were time-consuming, coupling reaction involving Cbz-Hyp(tBu)-Gly-OMe and Fmoc-Pro-OPf derivatives proved to be not efficient enough (giving the require product with 25% yield), the resulting products required purification steps by crystallisation. Low coupling reaction yields was most probably a consequence of tBu-type side chain protection for Hyp residue and can be explained by the steric hindrance and electronic properties of the fragment - Hyp(tBu)-Gly-OMe. The tBu is a group of choice for the protection of side chain of hydroxyproline residue, however is a barrier to facile and efficient peptide bond formation. The presence of the electron withdrawing group decreases the nucleophilicity of the α-amino group of the Hyp residue, in fact the coupling reactions with the hydroxyproline derivative (Hyp(tBu)) have been each time the lowest efficient steps, generating also difficult-to-purify by-products.

The coupling reaction of *N*-hydroxysuccinimide ester of *N*^{α}-protected dipeptide (Fmoc-Gly-Pro-OSu) with Hyp(tBu)-OH, produces mainly the formation of the cyclic dipeptide (diketopiperazine) and also tetrapeptide (Fmoc-Gly-Pro-Gly-Pro-OH), being a result of Fmoc deprotection/coupling reaction steps. Many undesired products such as diketopiperazines have been noticed in a particular case (if a cis peptide bond is formed by amino acid residues i.e. Gly, Pro).

Several attempts to eliminate the formation of diketopiperazines were made. The use of Cbz protecting group in side chain of Hyp prevented the steric hinderance, nevertheless it did not eliminate the formation of such undesired products. Mass spectrometry tools for the identification of product clearly indicated the peak of main product with a mass reduced by fragment of *N-*terminus of peptide (Gly-Pro), corresponding to the formation of diketopiperazine. These side reactions took place analogously during every Fmoc deprotection/acylation steps. In order to minimize the occurrence of such side reactions, Boc/Bzl strategy were preferred in the synthesis of different collagen fragments (i.e. during the formation of protected peptide chain Boc-Gly-Pro-Hyp(Bzl)-OH derivative), where every Boc deprotection occurs with TFA. The strategy was adopted to receive the fragments (units) of Pro-Pro-Gly, Pro-Hyp-Gly constituting the substrates in the synthesis of polymeric peptides. The method of obtaining a protected tripeptide (Cbz-Pro-Hyp-Gly-OBzl), wherein Cbz-Pro-Hyp-OH was coupled with H-Gly-OBzl using EDCI and HOBt, assumes then hydrogenolysis in the presence of *p*-toluenesulfonic acid and acylation with Fmoc-Su. The following reactions gave a good yield of the final product (Fmoc-Pro-Hyp-Gly-OH), preventing the formation of diketopiperazine. Nevertheless, the formation of *O*-acylated products, resulting from the use of coupling reactants such as HBTU and HOBt, can not be excluded. To prevent the formation of *O*-acylated products in the synthesis, the protected Hyp residue (Fmoc-Hyp(tBu)-OH) was always used.

### Treatment of chronic wounds

There still remains a need to develop a novel potential peptide-based drug in design to treatment of cardiovascular diseases or metabolic disorders i.e. diabetes mellitus. The incidence rate of diseases in a population increases by nearly 7% and 5% per year. Long-term complications include prolonged inflammation, tissue destruction, diabetic neuropathy, ischemia and infection that result in chronic or non-healing wounds.

Deep full thickness wounds, including also burn wounds (second or thrid degree), are a serious social problem with a high mortality risk. The wound healing is a particularly complex process, that requires the intricate synchronization of many elements, activation and coordination of series of biochemical and cellular events or processes. The growth factors are important for regulation certain types of cells and tissues (a key step during wound healing decides on the transition from the inflammatory phase to the proliferative phase).

Pathophysiological mechanisms of slow or non-healing wound progression are additionally implicated. Pro-inflammatory cytokine (TNF-α), which stimulates fibroblast and keratinocyte cells, contributes to the expression of growth factors and regulates the antimicrobial response in difficult-to-heal wounds or non-healing wounds, remains elevated. Oxidative stress can increase cytokine production, promoting inflammation and contributes to the impaired fibroblasts proliferation and decreasing the number of fibroblasts in extracellular matrix, thereby decreasing collagen secretion and protein concentration.

High glucose-induced expression of proinflammatory cytokine prompts both fibroblasts as well keratinocytes apoptosis. Apoptosis of fibroblasts was observed in humans in *in vivo* study as well with the use of mouse models during wound healing. However, TGF-β receptor expression is also reduced in chronic wounds, inhibiting of specialised cells (myofibroblast) conversion, that are required to organise wound edge contraction and extracellular matrix production. The impaired proliferative and migratory capacity of chronic wound cells is connected with the morphological changes and reduced expression of cell migration markers. Reconstructive transplantology is an effective strategy for proper treating second- or third-degree burns, but also venous ulcers, diabetic foot ulcers. Despite the advanced technologies to improve the management of diabetic foot ulcers, venous ulcers, pressure ulcers, they are still a challenge to wound care. Recent advances in the field of regenerative medicine and tissue engineering have resulted in the development of novel alternatives to transplantology. They are both the major challenges in the treatment of second- or third-degree burns, venous ulcers, diabetic foot ulcers. Tissue engineering and/or regenerative medicine are fields of life science employing both engineering and biological principles to create new tissues in therapy of extensive and hard-to-heal wounds. Therapy is based on design of scaffolds able to control cell activity (cell proliferation and differentation), components of the native ECM production. Biotechnological wound dressings or skin substitutes (tissue equivalents) are produced on an industrial scale, approved and currently used in the clinic.

Regenerative medicine using cultured epithelial cell grafts is a therapy of choice for deep partial and full-thickness burns (second and third degree burns). The first FDA approved product in 1996 for treatment of burns is extracellular matrix-based biomaterial called Integra ^{™} (a collagen/glycosaminoglycan scaffold). From patent description (WO99/63051) a method for producing a bi-layered bioengineered skin substitute (allogeneic skin) called Apligraf(Graftskin)^{™} (Organogenesis Inc., Novartis Pharmaceutical Corporation). In 1998 Apligraf(Graftskin)^{™} was registered and FDA approved for use in the treatment of venous ulcers and diabetic foot. The high cost of production as well as other requirements of these therapies prompt a search for new opportunities for wound therapies.

The discovery and development of topical medicines for wound healing have gained increasing attention. Most of current therapeutical agents applied for chronic wounds are growth factors, cytokines, chemokines, collagen and hyaluronic acid. Bekaplermin, a human platelet-derived growth factor BB that retains the activity of endogenous PDGF, manufactured by Johnson&Johnson, is available for treatment of diabetic neurophatic ulcers. European patent EP0575484 describes the manufacture and efficacy of a pharmaceutical composition containing PDGF. In turn, patent descriptions US6800286, US5155214 and US5981606 disclose the action of molecular growth factors i.e. FGF and TGF-β, which regulate various biological functions, including cell proliferation, survival, migration and differentiation. These therapeutical options include exept numerous advantages, a high production cost, reduced stability, short half-life, and other symptoms of topical application like erythema. Furthermore, most side effects of these therapies, (like excessive cell growth, psoriasis, impaired skin function and risk of cancer) have been observed at high concentrations level of growth factors. On the other hand, patent descriptions (US 2017/0007662 and WO 2014200651) indicate for short peptides derived from human C-X-C chemokines of the formula X₁-K-X₂, wherein X₁ is selected from amino acid residues i.e. Glu, Gln or Lys, X₂ is selected from Met, Phe, Ile, Trp, Val, being as multifunctional effector molecules responsible for stimulation of keratinocyte migration, neutralization bacterial pro-inflammatory factors and angiogenic effects on HUVECs. Reduction pro-inflammatory states by the aforementioned peptides was also investigated using *in vitro* full-thickness psoriasus skin model ( SOR-300-FT) Therefore, they are potential therapeutics in skin disease, including psiorasis, atopic dermatitis, rosacea. Another issue related to the use of novel compounds (substances) is drug delivery system, that requires a large amount of proteins for the biological effect. Many attempts has been made to use the polymer for extended release administration, improving the stability of the encapsulated proteins, however they are still constitute a challenge. Long-term release of growth factors remains difficult to control, requires the protein bioactivity during extended administration times. Although the bioactivity of proteins, growth factors is dependent on polymer incorporation method, it has not been thoroughly explored. Encapsulating proteins in polymer systems improves their stability, however, the modification of their structure may reduce their functionality.

Most of the work in novel wound therapy concentrates on the search of a biologically active compound, characterised by a low therapeutic index, a concentration-dependent action, devoid of any side effects, high selectivity.

### Technical problem:

Standard procedures for peptide synthesis, still require the modification and optimization to obtain the desired, therapeutical product, with increased efficiency, productivity and purity. In the reports described so far, the applied synthesis conditions, the methods for the formation of peptide bonds as well the selection of amino acid derivatives have become the most important to build the peptide chain rich with hydroxyamino acids. The Merrifield solid-phase technique in 1963 contributed to a huge progress in receiving the large biological molecules, overcoming the purification difficulties of intermediates resulted in classical solution-phase technique. However, efficient solid-phase-based total synthesis requires the appropriate conditions and production process optimization. Progress in chemical synthesis of peptides relies on modern coupling reagents, anti-racemization reagents, and other additives, that ultimately could contribute to increase the yield of resulting peptides-based compounds as well as the polycondensed products for medical or cosmetic purpose.

Convergent synthesis has been developed for the preparation larger or complex peptide/protein as well was used to prepare of collagen peptide fragments. The convergent strategy includes fragment condensation such as the solid- or solution-phase based protocols. Polycondensation of fully protected peptide fragments increases the purity and the yield of many final products. However, it requires to optimize the reaction conditions during synthesis hydroxy-amino acids rich peptides. However, the low efficiency of amino acids couplings and polycondensation of hydroxyamino acid-rich peptide fragments as well as difficult to separate by-products cause the overall low yield of the final products.

Hydroxyproline side chain protected by the most preferable protecting groups selectively removed during the synthesis, afforded the products in no relatively high yields. Despite the tactics of protection of side chain functions during solid-phase peptide synthesis in both Fmoc and Boc strategies, the invention discloses the manufacture procedures establishing a commercially available derivative of a hydroxyamino acid, i.e. Fmoc-Hyp-OH without protection of side chain (hydroxyl group). The method according to the invention provides specific sequences in high yields and high purity with the use of common reagents. The method was also adapted to polycondensation reactions according to the invention. The Fmoc-L-hydroxyproline proved to be resistant to *O*-acylation during amino acids coupling rections or peptide fragments polycondensation under the conditions applied according to the invention. The method of synthesis required optimization of each amino acid coupling step as well fragments polycondensation, in term of reagent selection i.e. coupling reagents, additives, time of the reaction and temperature. The method of synthesis according to the invention do not lead to the formation of other difficult to separate by-products.

### Aim of the invention:

The aim of the invention is to develop a rapid, efficient and scalable synthesis of hyroxy amino acid-rich lipopeptide conjugates, in particular hydroxyproline, applicable to receive highly pure novel lipopeptide conjugates and their pharmaceutically acceptable salts dedicated to use in a pharmaceutical composition or a preparation for stimulation of proliferation and migration processes of fibroblasts and keratinocytes, as a basic component for the manufacture of a wound dressing in a form of a solution, a suspension, an emulsion, an aerosol, a cream, an ointment, a three-dimensional scaffold, a hydrogel in a tube, applicable in burn wounds (second or third degree burns), venous leg ulcers, bedsores, or metabolic disorders i.e. diabetic foot, being a complication of existing cardiovascular disease or diabetes mellitus. The proposed method of manufacture ensures high safety of lipopeptide conjugates with their high biological activity.

### Disclosure of the invention:

The presented invention provides novel lipopeptide conjugates or pharmaceutically acceptable salts rich in Hyp residues located at positions AK₃, AK₄, which are known as mediators of tissue regeneration, by stimulating proliferation and migration processes of fibroblasts or keratinocytes. Peptide structures (consisted of less than 10 amino acid residue) show several advantages as novel and effective therapeutics. The high interest in the design and synthesis of peptide structures, applied in wound therapy, is their high specificity of action. However, due to their lower molecular weight and efficient degradability, they show a low toxicity profile and a better tissue penetration.

The object of our studies are collagen fragments and ECM degradation products as well proline-rich lipopeptide conjugates or lipopeptide conjugates composed of hydroxyamino acids, especially hydroxyproline residue, known as novel biologically active stuctures, involved in the wound healing process. Skin damage, characterised by a significantly impaired recovery process, prolonged inflammation associated with cardiovascular disease, diabetes mellitus, represents a critical stage for development of infections, tissue destruction, neuropathy, ischemia, mortality in patients after amputation. Therefore, essential efforts are being made to develop novel biologically active compounds, directly involve in the cellular processes in the second phase of wound healing, i.e. promoting proliferation, inducing a new tissue formation.

The disclosure of the invention is a novel L-hydroxyproline-rich lipopeptide conjugate of general formula:
*N*^{α}acyl-(AK₅-AK₄-AK₃-AK₂-AK₁)ₙ-NH₂ (I) or its pharmaceutically acceptable salt, wherein:
AK₁,AK₂,AK₃,AK₄,AK₅ denote amino acid residues or their lack;
AK₁ is the lysine residue of the L or D configuration.
AK₂ is an amino acid residue selected from the group consisting of non-polar neutral amino acids: Phe, Ala, Gly, Ile, Leu, Met, Pro, Val, Trp or its lack;
AK₃ is an amino acid residue selected from Hyp, Phe, Ala, Gly, Ile, Leu, Met, Pro, Val, Trp;
AK₄ is an amino acid residue selected from Pro, Hyp or its lack
AK₅ is the Gly residue;
*N*^{α}-acyl group has the formula R₁-C(=O), wherein R₁ is a saturated carbon chain of length from C₇ to C₁₃,
n denotes a number of repeat units from 1 to 2.

Advantageously, a lipopeptide conjugate according to the invention has an amide form of the conjugate or its pharmaceutically acceptable salt, the salt being selected from trifluoroacetate, acetate, hydrochloride, hydrobromide, hydroiodide, hydrofluoride, has the following sequences:

**Table 1**

| ***Lp.*** | ***N*^{α}-acyl** = **R₁-C(=O)** | **AK₅** | **AK₄** | **AK₃** | **AK₂** | **AK₁** | **AK₄'** | **AK₃'** | **AK₂'** | **AK₁'** | **n** |
|---|---|---|---|---|---|---|---|---|---|---|---|
| 1 | C₁₁ | Gly | - | Pro | - | L-Lys | - | - | - | - | 1 |
| 2 | C₁₁ | Gly | Hyp | Hyp | - | L-Lys | - | - | - | - | 1 |
| 3 | C₁₁ | Gly | Pro | Hyp | - | L-Lys | - | - | - | - | 1 |
| 4 | C₁₁ | Gly | Hyp | Ala | - | L-Lys | - | - | - | - | 1 |
| 5 | C₁₁ | Gly | Hyp | Hyp | - | D-Lys | - | - | - | - | 1 |
| 6 | C₁₁ | Gly | Hyp | Pro | - | L-Lys | - | - | - | - | 1 |
| 7 | C₇ | Gly | Hyp | Hyp | - | L-Lys | - | - | - | - | 1 |
| 8 | C₁₁ | Gly | Pro | Hyp | Gly | L-Lys | - | - | - | - | 1 |
| 9 | C₁₁ | Gly | Pro | Hyp | Met | L-Lys | - | - | - | - | 1 |
| 10 | C₁₁ | Gly | Pro | Hyp | Trp | L-Lys | - | - | - | - | 1 |
| 11 | C₁₁ | Gly | Hyp | Gly | Pro | L-Lys | - | - | - | - | 1 |
| 12 | C₁₁ | Gly | Pro | Hyp | Ile | L-Lys | - | - | - | - | 1 |
| 13 | C₁₁ | Gly | Pro | Hyp | Phe | L-Lys | - | - | - | - | 1 |
| 14 | C₁₁ | Gly | Pro | Hyp | Ala | L-Lys | - | - | - | - | 1 |
| 15 | C₁₁ | Gly | Pro | Hyp | Leu | L-Lys | - | - | - | - | 1 |
| 16 | C₁₁ | Gly | Hyp | Hyp | Val | L-Lys | - | - | - | - | 1 |
| 17 | C₁₁ | Gly | Hyp | Hyp | Pro | L-Lys | - | - | - | - | 1 |
| 18 | C₁₁ | Gly | Hyp | Leu | Pro | L-Lys | - | - | - | - | 1 |
| 19 | C₁₁ | Gly | Hyp | Met | Pro | L-Lys | - | - | - | - | 1 |
| 20 | C₁₁ | Gly | Hyp | Ala | Pro | L-Lys | - | - | - | - | 1 |
| 21 | C₁₁ | Gly | Hyp | Hyp | Gly | L-Lys | - | - | - | - | 1 |
| 22 | C₁₁ | Gly | Hyp | Hyp | Met | L-Lys | - | - | - | - | 1 |
| 23 | C₁₁ | Gly | Hyp | Hyp | Trp | L-Lys | - | - | - | - | 1 |
| 24 | C₁₁ | Gly | - | Hyp | Gly | L-Lys | - | - | - | - | 1 |
| 25 | C₁₁ | Gly | Hyp | Hyp | - | L-Lys | Pro | Hyp | - | L-Lys | 2 |
| 26 | C₁₁ | Gly | Hyp | Hyp | - | L-Lys | Hyp | Hyp | - | L-Lys | 2 |
| 27 | C₁₁ | Gly | - | Pro | - | L-Lys | Pro | Hyp | - | L-Lys | 2 |
| 28 | C₁₁ | Gly | - | Pro | - | L-Lys | Hyp | Hyp | - | L-Lys | 2 |
| 29 | C₁₁ | Gly | Pro | Hyp | - | L-Lys | Hyp | Hyp | - | L-Lys | 2 |
| 30 | C₁₁ | Gly | Pro | Hyp | - | L-Lys | Hyp | Hyp | - | D-Lys | 2 |
| 31 | C₇ | Gly | Pro | Hyp | - | L-Lys | Hyp | Hyp | - | L-Lys | 2 |
| 32 | C₉ | Gly | Pro | Hyp | - | L-Lys | Hyp | Hyp | - | L-Lys | 2 |
| 33 | C₁₃ | Gly | Pro | Hyp | - | L-Lys | Hyp | Hyp | - | L-Lys | 2 |
| 34 | C₁₁ | Gly | Pro | Hyp | - | L-Lys | Pro | Hyp | Gly | L-Lys | 2 |
| 35 | C₁₁ | Gly | Pro | Hyp | - | L-Lys | Hyp | Gly | - | L-Lys | 2 |
| 36 | C₁₁ | Gly | Pro | Hyp | - | L-Lys | - | Hyp | Gly | L-Lys | 2 |

The method of obtaining, on a polymeric carrier a lipopeptide conjugate of formula I, wherein n = 1, proceeds in the following steps:
(a) the coupling reaction of an active ester of Fmoc-L-Lys(Boc)-OH or Fmoc-D-Lys(Boc)-OH with an activated aminomethylpolystyrene proceeds via a 4-(2',4'-dimethoxyphenyl-Fmoc-aminomethyl)-phenoxyacetamide linker;
(b) attachment of the Fmoc-protected amino acid residues to obtain the sequence 1-24 according to Table 1 is carried out under anhydrous conditions, at 25±2°C for 3÷5 hours with DIPCI and HOBt at a molar ratio of 1:1:1 using 2.5÷5.0-fold excess of reactants in relation to the carrier loading in a NMP-DMF-DCM solvent mixture (1:1:1, v/v/v);
(c) attachment of a long chain fatty acid (C₈÷C₁₄) to an *N*^{α}-terminus of the peptidyl carrier is performed under anhydrous conditions at 25±2°C for 3÷5 h with TBTU, HOBt and DIPEA at a molar ratio of 1:1:1:2 using 2.5-fold excess of reactants in relation to the carrier loading in DMF with the addition of t-octylphenoxypolyethoxyethanol non-ionic surfactant (1%) (Cmc=267, HLB=14,4);
(d) cleavage of peptide conjugated with fatty acid from the polymeric carrier, defined by the sequence 1-24 as shown in Table 1, is carried out under inert gas, preferably nitrogen, in the dark for 2÷3 hours in a mixture of concentrated TFA, scavenger and/or antioxidant;
(e) purification of the cleaved lipopeptide conjugate is carried out by reversed-phase solid phase extraction (RP-SPE).

The method of obtaining on a polymeric support a lipopeptide conjugate of formula I, wherein n = 2, is carried out in parallel to obtain both two peptide chains A and B on a 2-chlorotrityl carrier (fragment A) and aminomethylpolystyrene (fragment B), respectively, in the following reactions:
(a) coupling reaction of an active ester of Fmoc-L-Lys(Boc)-OH or Fmoc-D-Lys(Boc)-OH with an activated polymeric carrier, which is polystyrene having as linker a 2-chlorotrityl ester (fragment A) and aminomethylpolystyrene having as linker 4-(2',4'-dimethoxyphenyl-Fmoc-aminomethyl)-phenoxyacetamide (fragment B), respectively;
(b) attachment of the Fmoc protected amino acid residues to obtain the peptide fragment A and peptide fragment B is carried out under anhydrous conditions, at 25±2°C for 3÷5 hours with DIPCI and HOBt in a molar ratio 1:1:1 using 2.5÷5.0 fold excess of reactants in relation to the carrier loading, in a NMP-DMF-DCM solvent mixture (1:1:1, *ν*/*ν*/*ν*);
c) attachment of a long-chain fatty acid (C₈÷C₁₄) to an *N*^{α}-terminus of the peptidyl carrier (fragment A loaded carrier) is performed under anhydrous conditions, at 25±2°C for 3÷5 hours with TBTU, HOBt in the presence of DIPEA in the molar ratio 1:1:1:2 using 2.5-fold excess of reactants in relation to the carrier loading, in DMF with the addition of t-octylphenoxypolyethoxyethanol non-ionic surfactant (1%) (Cmc=267, HLB=14,4);
(d) cleavage of the lipopeptide chains (lipo-fragment A) from the polymeric carrier by treatment of the carrier with DCM-TFA (95:5, *ν*/*ν*) or DCM-TFA-iPr₃SiH (90:5:5, *ν*/*ν*/*ν*) in an inert gas, preferably nitrogen, in the dark for 30 minutes, followed by neutralisation of the cleaved lipopeptide chains and purification,
(e) polycondensation reaction of peptide fragments: purified lipo-fragment A and fragment B loaded aminomethylpolystyrene carrier is performed under anhydrous conditions at 25±2°C for 2.5÷5.0 h using TBTU, HOBt in the presence of DIPEA in the molar ratio 1:2:2:4, using 2.5÷8.0 fold excess of reactants in relation to the carrier loading, in DMF;
(f) cleavage of the lipopeptide conjugate defined by the sequence 25-36 according to Table 1 from the carrier is performed under an inert gas atmosphere, preferably nitrogen, in the dark, for a period of 2÷3 hours in the presence of a mixture of concentrated TFA, scavengers and/or antioxidant;
(g) purification of the cleaved lipopeptide conjugate is carried out by reversed-phase solid phase extraction (RP-SPE).

Advantageously, the method of obtaining wherein loading of the first amino acid viz. Fmoc-L-Lys(Boc)-OH or Fmoc-D-Lys(Boc)-OH on the 2-chlorotrityl carrier and aminomethylpolystyrene carrier is in the ranges of (0.655÷1.245)±0.040mmol/g, and (0.345÷0.790) ±0.050mmol/g, respectively.

Advantageously, the hydroxyproline-rich lipopeptide conjugates described in general formula I according to the invention are obtained using Fmoc-*N*-protected hydroxyamino acid, i.e. Fmoc-L-4-hydroxyproline.

Advantageously, the coupling reaction between the free α-amino group of the amino acid residue in the sequence of the L-4-hydroxyproline-containing peptidyl carrier and the carboxyl group of the successively coupled Fmoc-protected amino acid residue to build hydroxyproline-rich lipopeptide conjugate of general formula I, is carried out using Fmoc-protected amino acid (Fmoc-AK-OH), DIPCI and HOBt in a molar ratio of 2:2:2 in a mixture of NMP, DMF and DCM in a volume ratio of 1:1:1, at 18±2°C, for 3.5±0.5 hours.

Advantageously, a method of obtaining the L-hydroxyproline-rich lipopeptide conjugates wherein they are cleaved from the aminomethylpolystyrene carrier using a mixture of concentrated TFA and scavengers i.e. PhOH, iPr₃SiH, H₂O in a volume ratio of 88:5,8:2:4.2 over a period of 2÷3 hours, at 25±2°C or a mixture of concentrated TFA and scavengers i.e. PhOH, iPr₃SiH, H₂O with the addition of NH4I, in a volume ratio of 86.5:5.8:4.2:1.5 for a period of 3÷4 hours, preferably at 0°C.

Advantageously, a method of obtiaining the lipopeptide conjugates, wherein they are cleaved from the polymeric carrier according to the invention establishes a purification process by RP-SPE, using columns packed with the octadecyl group bonded type silica gel with gradient elution at concentrations in the range from 0% to 30% acetonitrile in water with the addition of 0.1% trifluoroacetic acid (TFA), and isocratic elution.

A pharmaceutical composition according to the invention comprises at least one lipopeptide conjugate or pharmaceutically acceptable salt defined by the sequence 1-36 according to Table 1 in the amount varies from 0.0001% to 10% w/w and at least one pharmaceutically acceptable solvent. A preparation for stimulating proliferation and migration of fibroblasts and keratinocytes comprises a pharmaceutical composition according to the invention in the amount varies from 0.01% to 10% by weight, of the total weight of a wound dressing.

A pharmaceutical composition as defined above comprising a lipopeptide conjugate or a pharmaceutically acceptable salt as defined above, or a preparation according to the invention, are used as a basic component for the manufacture of a wound dressing, in the form of a solution, a suspension, an aerosol, an emulsion, a cream, an ointment, a three-dimensional scaffold, or a hydrogel in a tube.

A pharmaceutical composition as defined above comprising a lipopeptide conjugate or a pharmaceutically acceptable salt as defined above, or a preparation as defined above, or a wound dressing according to the invention are used for the treatment of chronic wounds, advantageously burn wounds, venous leg ulcers bedsores, diabetic foot, as well as those which are a complication of existing cardiovascular diseases or diabetes mellitus.

The biological activity of a pharmaceutical composition comprising a novel hydroxyproline-rich lipopeptide conjugate or a pharmaceutically acceptable salt, or a preparation or wound dressing, was determined in preclinical studies, both *in vitro* and *in vivo.* Example 7 defines the ability of a pharmaceutical composition comprising a novel hydroxyproline-rich lipopeptide conjugate or a pharmaceutically acceptable salt thereof according to the invention to initiate and modulate cell migration rates and reduce wound surface area. A pharmaceutical composition comprising a novel hydroxyproline-rich lipopeptide conjugate or a pharmaceutically acceptable salt or a preparation obtained according to the invention was tested using the method described in Example 7.

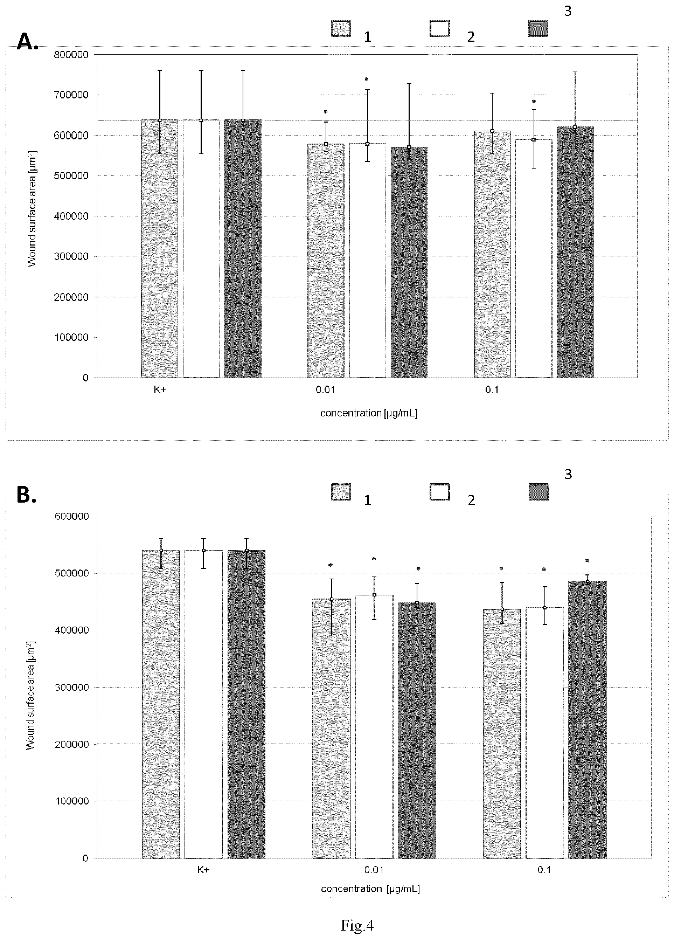

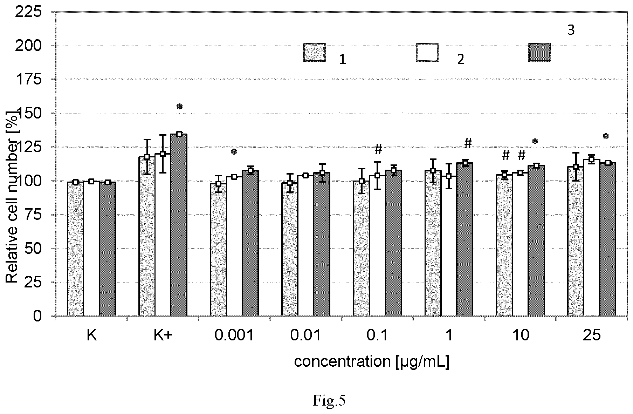

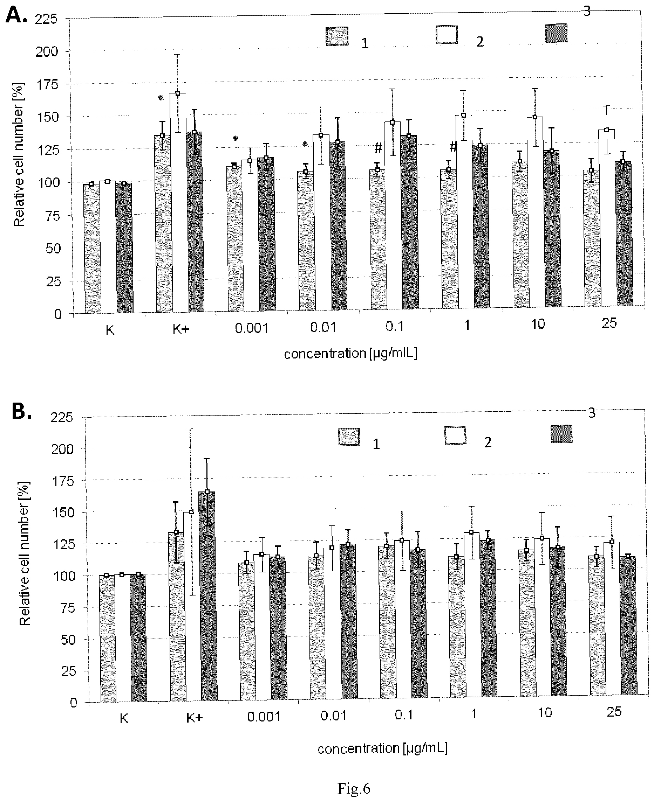

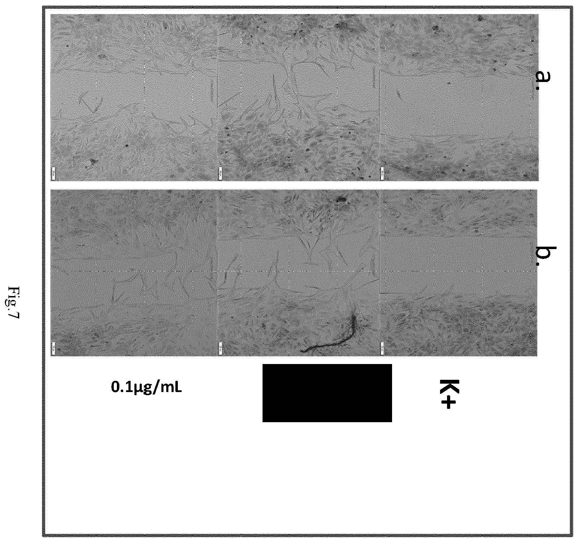

Microscopic images shows a primary fibroblast cells migration in wound healing assay. The result of examination of wound closure with pharmaceutical composition comprising a novel hydroxyproline-rich lipopeptide conjugate or a pharmaceutically acceptable salt thereof at a given concentration are shown in Figures 4 and Figures 7. The role of the pharmaceutical composition containing the novel hydroxyproline-rich lipopeptide conjugate or its pharmaceutically acceptable salt or a preparation in modulating the rate of cell migration was observed, possibly in concentration-dependent manner as well as connected with specific amino acid sequence. However, cell proliferation activity was simultaneously assessed by the method described in Example 8. The effect of the concentration on the relative number of cells treated with a pharmaceutical composition containing a novel hydroxyprline-rich lipopeptide conjugate or a pharmaceutically acceptable salt are shown in Figure 5, Figure 6 and Table 4.

Hyp-rich lipopeptide conjugates or their pharmaceutically acceptable salts provided an optimal environment for the growth, development and viability of skin cells. High HaCaT cell activity, as well as a significant activation of the fibroblast proliferation process (i.e. transformed human fibroblast cells as well primary cultured fibroblasts derived from patients) in the presence of a pharmaceutical composition containing a lipopeptide conjugate or its pharmaceutically acceptable salt was determined in a wide range of concentrations.

Hyp-rich lipopeptide conjugates or pharmaceutically acceptable salts obtained according to the invention illustrated in examples from 1 to 6 present distinguishing features from others arising from the specific action They present a high safety profile, toxicity evaluation was based on the hemolysis, enzyme activity. *In vitro* hemolytic activity was determined by general method described in Example 10.

Table 6 shows the results from the measurements. The HC₁₀ values of the lipopeptide conjugates or their pharmaceutically acceptable salts varied in the concentration range of 0.19÷1.62mM. No hemolytic effect causing 50% hemolysis of cells (HC₅₀) was observed in the whole tested concentration range, providing their high safety, biocompatibility and protective effect against cells. Compounds-induced toxicity in human skin cells was also measured using the LDH, XTT assays described in Example 9. Table 5 lists the values of EC₁₀ and EC₅₀ for lipopeptide conjugates or their pharmaceutically acceptable salts according to the invention. The results confirmed the preclinical safety profile of lipopeptide conjugates or their pharmaceutically acceptable salts over the range of their biological action.

To date, no other relevant pharmaceutical composition comprising a lipopetide conjugate or a pharmaceutically acceptable salt or a preparation obtained according to the invention are available commercially and in clinical care of skin wound healing. The combinations of multiple peptide-based therapeutics and polymeric matrices and various peptide fragments has been reported in preclinical studies. However, only a topical ACT1 peptide (RQPKIWFPNRRKPWKRPRPDDLEI) embedded in a hydroxyethyl-cellulose- based hydrogel called Granexin^{®} has entered phase II clinical trial (2015). Such combinations containing innovative lipopeptide conjugates according to the invention are directed to new generation products formation, i.e. a wound-dressing in the form of a solution, a suspension, an emulsion, an aerosol, a cream, an ointment, a three-dimensional scaffold, a hydrogel in a tube, applicable in chronic, non-healing wounds associated with systemic diseases of metabolic and venous origin.

The pharmaceutical composition comprising a lipopeptide conjugate or a pharmaceutically acceptable salt or a preparation according to the invention, produced by the method according to the invention, may be topically administered in this case in a therapeutically effective amount. A pharmaceutical composition comprising a lipopeptide conjugate or a pharmaceutically acceptable salt or a preparation according to the invention may be used in a wound-dressing in the form of a solution, a suspension, an emulsion, an aerosol, a cream, an ointment, a three-dimensional scaffold, an amorphous hydrogel extruded from a tube.

Hydrogel dressings contain a polymeric matrix, in particular cellulose derivatives (MC, HPMC, CMC, HPC), but also could contain other pharmaceutically and cosmetically acceptable ingredients. Water, buffers, especially phosphate buffers, saline, or other physiologically balanced salt solutions can be used as pharmaceutically acceptable solvents.

Other substances used in the preparation of emulsions, creams, ointments constitute the oil phase. The preparations and wound-dressings prepared according to the invention may be liquid or solid, the pharmaceutical compositions containing lipopeptide conjugates or their pharmaceutically acceptable salts are administered as a suspension or a solution in a therapeutically effective amount from 0.0001% to 10% (w/w). In one embodiment of the invention, the pharmaceutical composition containing at least one lipopeptide conjugate or pharmaceutically acceptable salt comprises a carboxymethylcellulose derivative as a polymeric matrix for controlled release.

The pharmaceutical composition comprising a lipopeptide conjugate or its pharmaceutically acceptable salt or a preparation is applied to the wound bed followed by protection with an occlusive dressing fixed tape bandages to prevent infection and other risk factors, i.e. contamination, trauma. The pharmaceutical composition or preparation according to the invention may be administered in therapeutically effective amounts treatment schedule-dependent. Treatment plan (an appropriate amount to be given, schedule of administration) can be estimated on the basis of the presented experimental results (the relationship between therapeutical effect and an amount of the pharmaceutical composition containing the lipopeptide conjugate or its pharmaceutically acceptable salt or a preparation).

The examples illustrate the use of a pharmaceutical composition comprising a lipopeptide conjugate or a pharmaceutically acceptable salt or a preparation in a therapeutically effective amount from 0.1% to 4.0% w/w. The pharmaceutical composition or preparation is administered to the wound area with standard equipment for topical application. The cellulose derivatives have the potential to be used as drug-delivery material due to high chemical compatibility with lipopeptide conjugates and their pharmaceutically acceptable salts according to the invention. Formulations based on carboxymethylcellulose (CMC) derivatives (the main component of commercially available next generation wound-dressing i.e. Granugel^{®} (ConvaTec), Intrasitegel^{®} or Solositegel^{®} (Smith&Nephew)) are characterised by desirable physical properties, controlled release capabilities as well as different viscosities. In one embodiment of the invention, the wound-dressing composed of a pharmaceutical composition comprising
a lipopeptide conjugate or a pharmaceutically acceptable salt or a preparation in an amount in the range from 0.01% to 5% by weight and carboxymethylcellulose in an amount in the range of 1% to 20%.

In another form of the invention, a cream or an emulsion in areosol produced by conventional pharmaceutical and cosmetic techniques, comprises an emulsifying system and a pharmaceutical composition or preparation in an amount in the range of 0.2÷10% by weight of the emulsion weight.

The emulsifying system advantageously comprises the following ingredients, i.e. solid lipids (emollients 1÷10% i.e. lanolin, fatty alcohols (i.e. cetyl, stearic)), 1÷10% surfactant (emulsifier), however an aqueous phase composes of humectants preferentially from 1 to 15% (i.e. 2÷10% glycerin, propylene glycol, butylene glycol), a preservative, i.e. 0.1÷2% phenoxyethanol, methyl ester of 4-hydroxybenzoic acid, ethyl ester of 4-hydroxybenzoic acid and up to 100% distilled water. Preclinical tests have shown that pharmaceutical compositions or preparations or wound dressings containing lipopeptide conjugates or pharmaceutically acceptable salts improve wound healing outcomes in diabetic foot ulcers. A pharmaceutical composition containing a lipopeptide conjugate or its pharmaceutically acceptable salt or a preparation or a next-generation wound-dressing is directly involved in the construction of new tissue through granulation and epithelialization. Treatment of specific types of wound with a pharmaceutical composition containing a lipopeptide conjugate or a pharmaceutically acceptable salt, or a preparation or a wound-dressing, is carried out for a certain period, depending on disease severity and condition of the wound. The wound dressing application is more comfortable that is changed on every 2÷3 days.

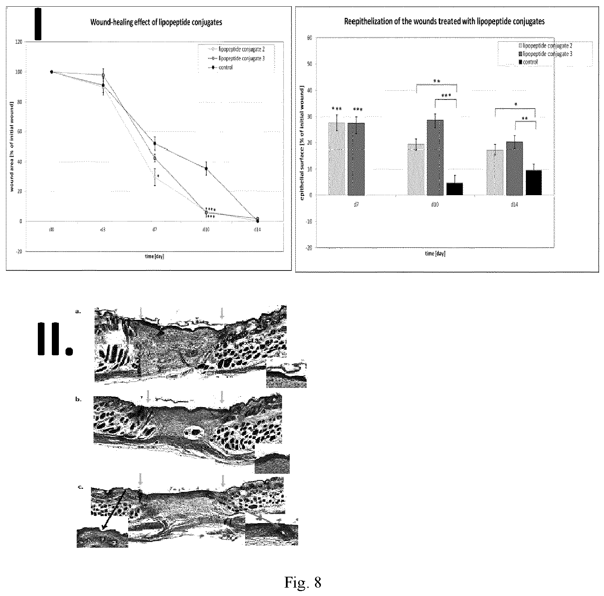

The efficacy of pharmaceutical compositions or preparations or wound-dressings manufactured according to Example 11 was proved by evaluating the ability to activate the wound healing process *in vivo* as described in Examples 13, 14. The results showing their effect on wound-healing and reconstruction of full-thickness skin in BALB/c and db/db mice over a period of several days treatment with a pharmaceutical composition containing a lipopeptide conjugate or a pharmaceutically acceptable salt thereof or a preparation or a wound-dressing are disclosed in FIGS. 8÷17.

The novel lipopeptide conjugates of the invention are involved in the second phase of wound-healing i.e. proliferation phase, an essential step stimulating the new tissue formation (newly granulation tissue formation, re-epithelialization, wound closure). As shown, novel lipopeptide conjugates or their pharmaceutically acceptable salts are able to initiate and stimulate *in vitro* directional cells movement (chemotaxis) and development and growth. Experimental studies demonstrated their directly action on major cells (fibroblasts) responsible for the production of collagen and a major components of ECM.

### Figure and table description:

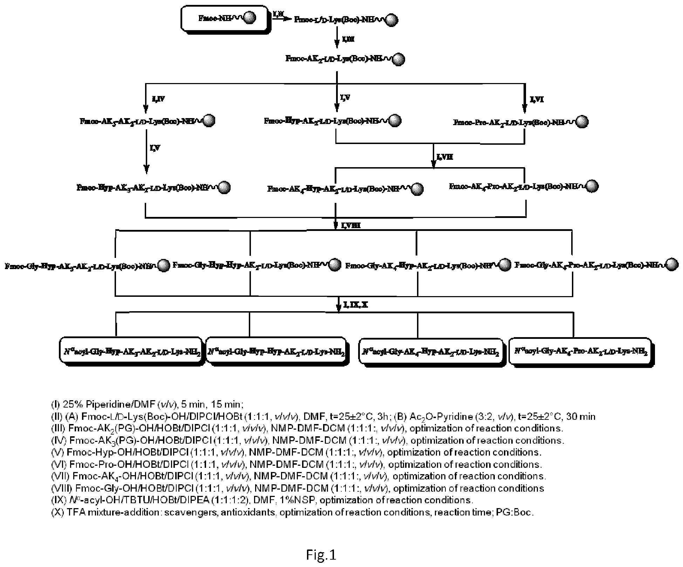

**Fig.1** Schematic illustration of solid-phase synthesis of lipopeptide conjugates corresponding to the formula I (n=1) on aminomethyl polystyrene carrier.

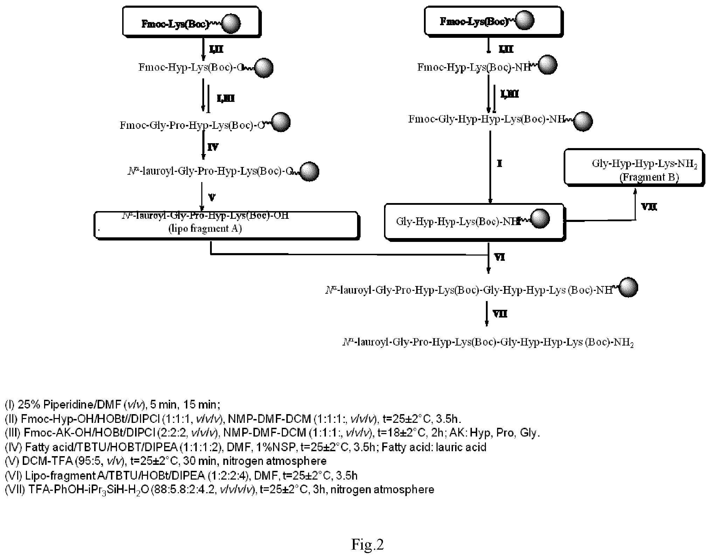

**Fig.2** Schematic representation for the synthetic strategy of obtaining *N*^{α}-*lauroilo*-Gly-Pro-Hyp-Lys-Gly-Hyp-Hyp-Lys-NH₂ by the (poly)condensation of *N*^{α}-acylpeptide (Lipo-fragment A) with α-amine group of peptidyl fragment (Fragment B) supported on aminomethylpolystyrene carrier.

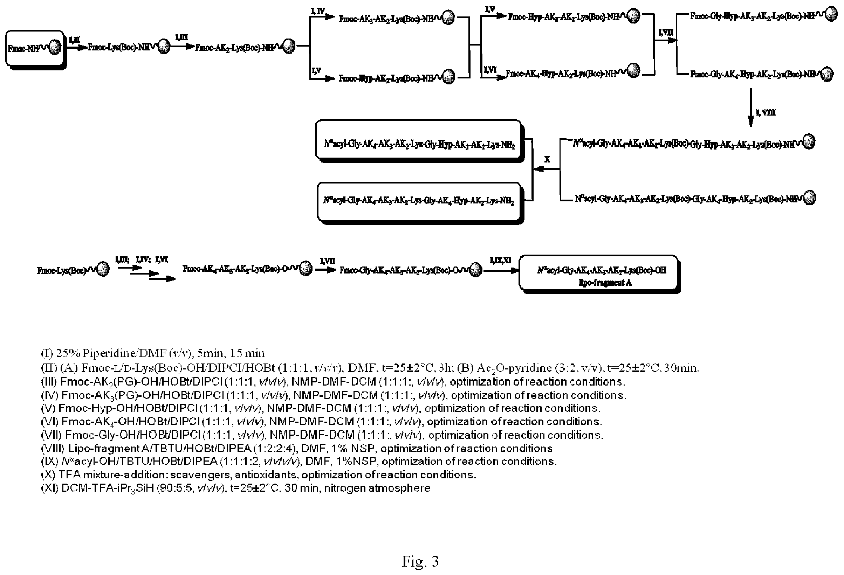

**Fig.3** Schematic illustration of solid-phase synthesis of lipopeptide conjugates corresponding to the formula I (n=2) on aminomethyl polystyrene carrier.
**Fig.4** Analysis of migration capacity of human skin cells treated with pharmaceutical composition (water solution) comprising lipopeptide conjugate 1 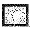 , 2 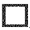 , 3 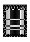 in a concentration of 0,01µg/mL and 0.10µg/mL. The cell migration capacity was assessed using wound healing assay by IBIDI Culture-inserts as described in Section 2. Results are presented as the median and min-max, n=3; *statistical difference in relation to control p<0.05. K+ positive control in DMEM medium, human fibroblast cell line (BR1N), B. primary fibroblasts.
**Fig.5** The effect of pharmaceutical composition (water solution) comprising lipopeptide conjugate 1 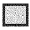 , 2 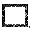 , 3 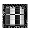 in a concentration range of 0.001÷25µg/mL on the proliferation of human skin cells: primary fibroblasts. K, control in DMEM, K+, positive control, in DMEM medium. The growth of cells was estimated by XTT test as described in Section 2. Results are presented as the mean±SD, n=3. Statistical differences in relations to control (K) are indicated as ^{∗}p <0.05, ^{∗∗}*p*<0.01. Statistical differences in relations to positive control (K+) are indicated as ^{#}p<0,05.
**Fig.6** The effect of pharmaceutical composition (water solution) comprising lipopeptide conjugate 1 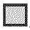 , 2 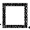 , 3 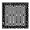 in a concentration range of 0.001÷25µg/mL on the proliferation of human skin cells: A. HaCaT keratinocyte, B. human fibroblast cell line BR1N. K, control in DMEM, K+, positive control, in DMEM medium. The growth of cells was estimated by XTT test as described in Example 8 (Examples of application). Results are presented as the mean±SD, n=3. Statistical differences in relations to control (K) are indicated as ^{∗}p <0.05, ^{∗∗}*p*<0.01. Statistical differences in relations to positive control (K+) are indicated as ^{#}p<0,05.
**Fig.7****.** Microscopic images of primary fibroblast cells migration in wound healing assay. Examination of wound closure with pharmaceutical composition (water solution) comprising lipopeptide conjugate: a. lipopeptide conjugate 1, b. lipopeptide conjugate 2 in a concentration of 0.01µg/mL and 0.10µg/mL. K+, positive control, in DMEM medium supplemented with FCS.
**Fig.8****.** The wound-healing effect of a preparation (0.1%w/w). Quantification of the remaining wound area and epithelial area at different time points after wounding Wound area measured and expressed as a percent of wound area at Day 0 in the lipopeptide conjugate-treated group n (number of wounds) =12 and in the FA₄ - treated control group n (number of wounds) =12. From Day 3 in lipopeptide conjugate-treated animals significantly accelerated wound reepithelization. Results are presented as the mean±SEM, n=3. Statistical difference in relation to control are indicated as *^{∗}p*<0.05, ^{∗∗}*p*<0.01, ^{∗∗∗}*p*<0,001. Microscopic images of Masson's trichrome-stained sections of BALB/c mice skin treated with lipopeptide conjugate 2 (a), lipopeptide conjugate 3 (b), control samples of untreated healthy skin (c) given after a period of 18 days. Gray arrows point the wound margin. Gray and black arrows indicate the newly formed epidermis on Day 18. Masson's trichrome stained section. Masson's trichrome stain indicated the presence of collagen and elastin fibres and restoration of normal skin architecture during wound healing (dermis with indistinguishable boundaries between host and regenerated skin).
   Wound reepithelialization in BALB/c mice 18 days after injury. The section of mouse skin treated with lipopeptide conjugate 2 revealed thickening of the epidermal layer compared to the normal skin (c). The increase in epithelium thickness is a result of wounding and rise in the number of keratinocytes (cell proliferation).

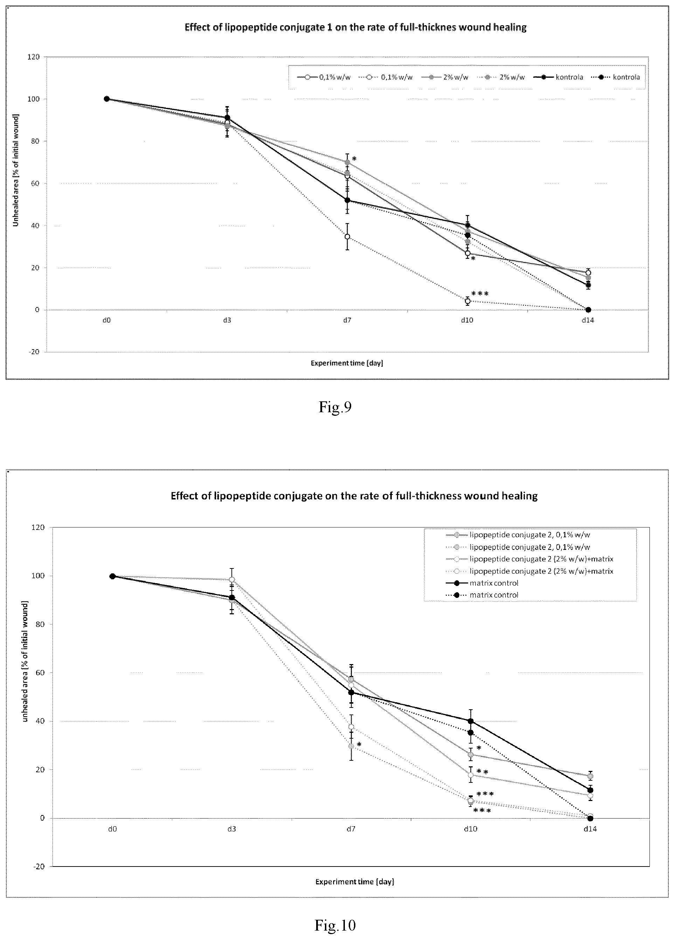

**Fig.9** - Scheme of administration and the effect of different concentration of lipopeptide conjugate 1 on the rate of full-thickness wound healing in BALB/c mice. Diffrerences in wound size reduction and coverage of the wound with a new epithelial layer are visible from Day 3. Application of a 0.1% w/w preparation to wounds (n ( number of wounds) = 12) took place every day (d0÷d10), however the application of a 4.0% w/w preparation took place at regular time intervals d0, d3, d7, d10 (4 times during the 18-day treatment). The restoration of full-thickness skin is determined by the reduction in the surface area of wound over time (solid line is show on the graphical presentation). The area of the newly formed epithelial layer/epidermis in wound over time is defined in relation to the wound surface area over time (dashed line). Results represent the mean value ± SEM. Statistical differences in relations to control (next generation hydrogel dressing (FA4), n=10÷12 (d10,d14)) are indicated as ^{∗}p<0.05, ^{∗∗}p<0.01, ^{∗∗∗}p<0.001).
**Fig.10** - The effect of lipopeptide conjugate 2 after its application on full-layered wounds in BALB/c mice. Application of a preparation in a therapeutical amount of 0.1÷4.0%, w/w. The use of a pharmaceutical composition in the polymerix matrix - sodium carboxymethylcellulose in an amount of 4.0% w/w allowing the free release of the lipopeptide conjugate from matrix during the time intervals of experiment (d0, d3, d7, d10). Differences in wound size and coverage of the wound with a new epithelial layer are visible from Day 3. Application of a 0.1% w/w preparation to wounds (n (number of wounds) = 12) took place every day (d0 ÷ d10), however the application of a 4.0% w/w preparation took place at regular time interwal d0, d3, d7, d10 (4 times during the 18-day treatment). The restoration of full-thickness skin is determined by the reduction in the surface area of wound over time (solid line is show on the graphical presentation). The area of the newly formed epithelial layer/epidermis in wound over time is defined in relation to the wound surface area over time (dashed line). Results represent the mean value ± SEM. Statistical differences in realations to control of the polimeric matrix, n = 10-12 are indicated as ^{∗}p<0.05, ^{∗∗}p<0.01, ^{∗∗∗}p<0.001).

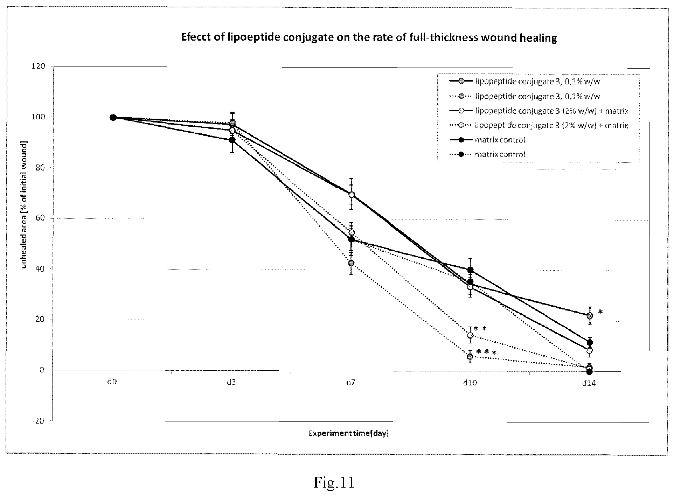

**Fig.11** - The effect of lipopeptide conjugate 3 after its application on full-layered wounds in BALB/c mice. Application of a preparation in a therapeutical amount of 0.1÷4.0%, w/w. Differences in wound size and coverage of the wound with a new epithelial layer are visible from Day 3. Application of a 0.1% w/w preparation to wounds (n (number of wounds) = 12) took place every day (d0÷d10), however the application of a 4.0% w/w preparation took place at regular time intervals d0, d3, d7, d10 (4 times during the 18-day treatment). The use of a pharmaceutical composition in the polymer matrix - sodium carboxymethylcellulose in the amount of 4.0% w/w allowing the free release of the lipopeptide conjugate from matrix during the time intervals of experiment (d0, d3, d7, d10). The restoration of full-thickness skin is determined by the reduction in the surface area of wound over time (solid line is shown on the graphical presentation). The area of the newly formed epidermie in wound over time is defined in relation to the wound surface area over time (dashed line). Results represent the mean value ± SEM. Statistical differences in relations to control of the polymeric matrix, n = 10-12 are indicated as ^{∗}p<0.05, ^{∗∗}p<0.01, ^{∗∗∗}p<0.001).

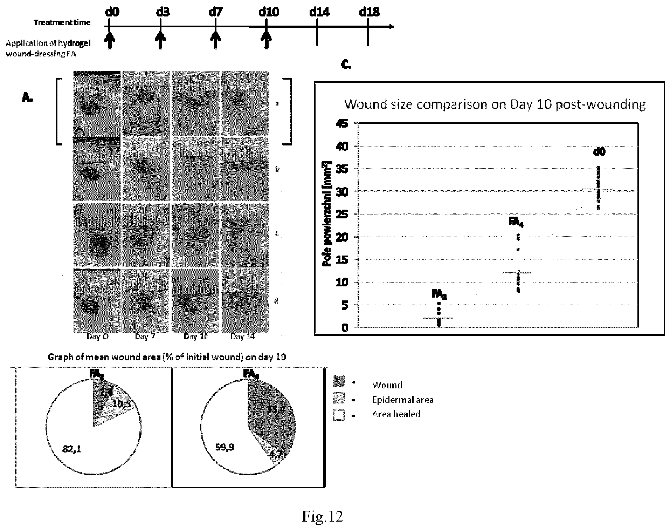

**Fig.12** - Effect of stimulation of wound healing process in mice with preparations containing lipopeptide conjugates. A. Macroscopic evaluation of wound closure in BALB/c mice under treatment with hydrogel wound dressing. Effect of stimulation of wound healing process in mice with the preparations containing lipopeptide conjugates. A. Macroscopic evaluation of wound closure in BALB/c mice under treatment with hydrogel wound dressings. Photographs of wounds were taken from each Mouse in the group treated with FA₁ (b), FA₂ (c), FA₃ (d) compared to control FA₄ (a). Wounds were made on the dorsal skin site on Day O. B. Graph of mean wound area (% of initial wound), epidermal area or area healed on Day 10 treated with FA₂ (left) versus FA₄ (right). C. The wound area on Day 10 after treatment with FA₂ compared to FA₄.

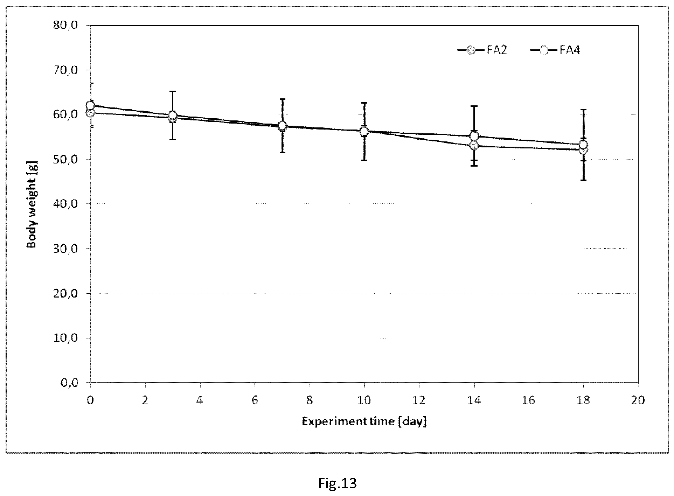

**Fig.13** - The changes in body weight of female db/db mice under treatment of wounds with FA₂ and FA₄. Points represent mean value ± SD (n = 5). Statistical differences in relations to control (FA₄) are idicated as ^{∗} p <0.05.

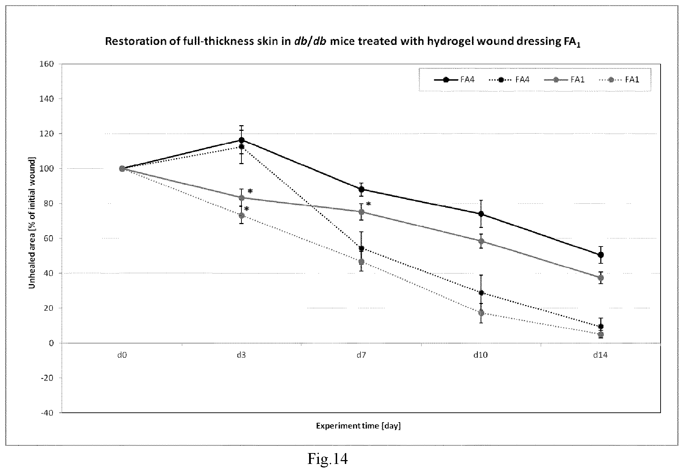

**Fig.14** - Regeneration of mice skin tissue induced by treatment with FA₁. The restoration of full-thickness skin is determined by the reduction in the wound surface area over time (n (number of wounds) = 10) (control (FA₄), n=12-14)) (solid line is shown on the graphical presentation). Wound area (% of initial wound (on Day 0)) indicated a progressive reepithelization process from Day 3 after treatment with FA₁ (dashed line). Results represent the mean value ± SEM. Statistical differences in relations to control (FA₄) are indicated as ^{∗}p<0.05.

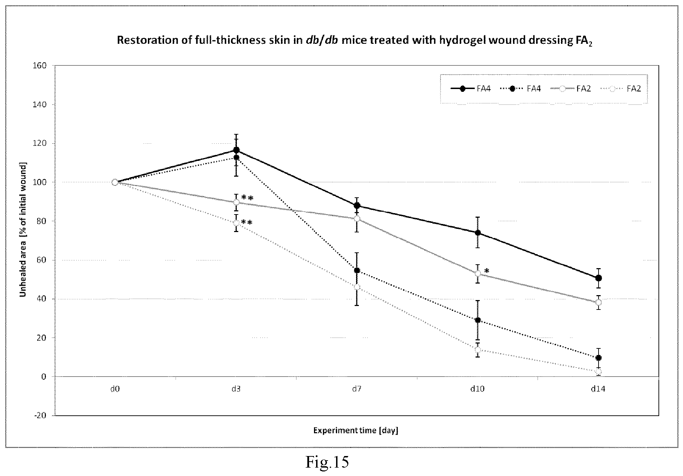

**Fig.15** - Regeneration of mice skin tissue induced by treatment with FA₂. The restoration of full-thickness skin is determined by the reduction in the wound surface area over time (n(number of wounds) = 12-16) (control (FA₄), n=12-14))
   (solid line is shown on the graphical presentation). Wound area (% of initial wound (on Day 0)) indicated a progressive reepithelization process from Day 3 after treatment with FA₂ (dashed line). Results represent the mean value ± SEM. Statistical differences in relations to control (FA₄) are indicated as ^{∗}p<0.05.

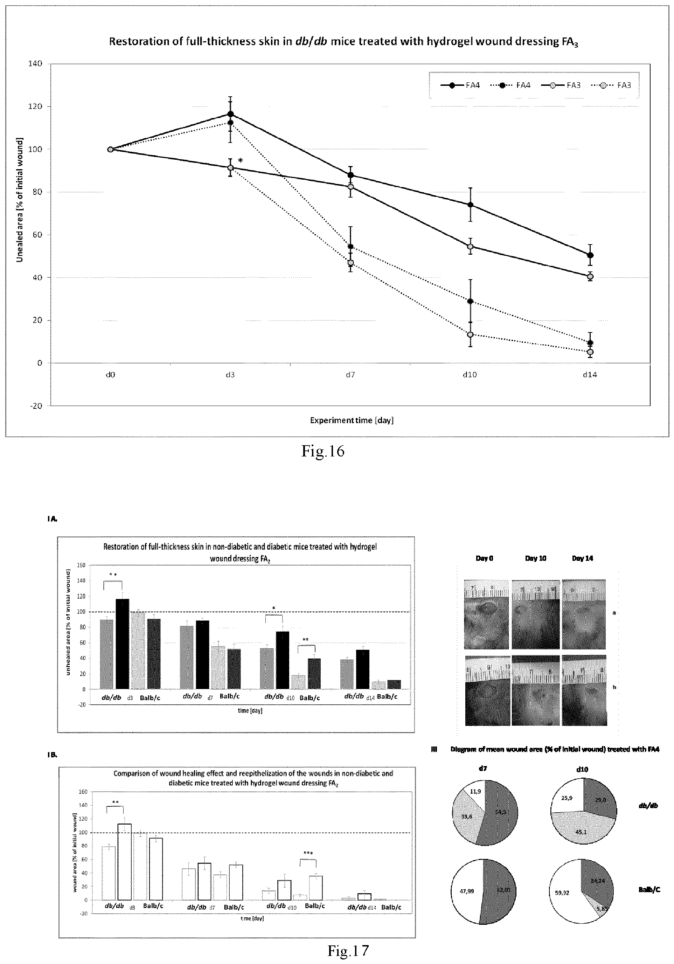

**Fig.16** - Regeneration of mice skin tissue induced by treatment with FA₃. The restoration of full-thickness skin is determined by the reduction in the wound surface area over time (n(number of wounds) = 8-10) (control (FA₄), n=12÷14))
   (solid line is shown on the graphical presentation). Wound area (% of initial wound (on Day 0)) indicated a progressive reepithelization process from Day 3 after treatment with FA₃ (dashed line). Results represent the mean value ± SEM. Statistical differences in relations to control (FA₄) are indicated as ^{∗}p<0.05.
**Fig.17** - The effect of applied hydrogel wound dressing FA₂ on full-layered wounds in both non-diabetic BALB/c mice and *db*/*db* mice. Wounds of BALB/c (n =12÷16) and db/db (n=6/8) mice were treated with FA₂ at d0, d3, d7, d10, the area of an open wound covered with new epithelium was measured. IA. The restoration of full-thickness skin is determined by the reduction in the surface area of wound during the time intervals of experiments (d3, d7, d10, d14) ( 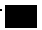 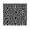 FA₄ 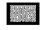 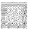 FA₂). IB. The area of newly fomed epithelial layer/epidermis over time is determined in relation to the area of the remaining wound over time ( 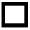 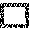 FA₄ 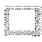 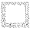 FA₂). On Day 18, the wounds were completely healed relative to the control (FA₄). Results represent the mean value±SEM. Statistical differences in relations to control are indicated as ^{∗}p<0.05, ^{∗∗}p<0.01, ^{∗∗∗}p<0.001). II. A. Macroscopic evaluation of wound closure in *db*/*db* mice under treatment with hydrogel wound dressings as exemplified by FA₂. Photographs of wounds were taken from each mouse in the group treated with FA₂ (a) compared to the control FA₄, (b). Wounds were made on the dorsal skin site on day 0.
   III. Graph comparing the values of mean wound areas in non-diabetic mice and with advanced diabetes mellitus (% of initial wound) 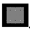 , epidermal area 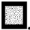 , area healed 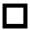 on day 7, 10, treated with FA₄.

**Table 1.** Series of lipopeptide conjugates corresponding to the formula I
**Table 2.** Comparison of different reaction conditions between free α-amine group of Hyp-rich peptide loaded aminomethylpolystyrene carrier and carboxylic group of the next Fmoc-protected amino acid (Fmoc-AA-OH) to build desire lipopeptie conjugate.
**Table 3.** Scalability of effective procedure (Method 1) for the synthesis of lipopeptide conjugates loaded aminomethylpolystyrene carrier.
**Table 4.** Wound closure with human keratinocyte primary culture induced by a pharmaceutical composition (water solution) containing lipopeptide conjugate.
**Table 5.** Effective concentration of lipopeptide conjugates causing 10% and 50% decrease in the viability of human skin cells.
**Table 6.** Hemolytic activity of lipopeptide conjugates in human erythrocytes; HC₁₀, HC₅₀ are defined as the lowest concentration of lipopeptide conjugate induced 10% and 50% cell lysis; median, 25÷75%; nd - 50% cell lysis was not determined after the application of lipopeptide conjugate, ^{∗}10÷500 µg/mL.
**Table 7.** Example of hydrogel composition
**Table 8.** Composition of an o/w spray emulsion (FB) prepared on the basis of US200502555048, additionally containing lipopeptide conjugates.
**Table 9.** Composition of an o/w emulsion (FC), additionally containing lipopeptide conjugates.

The invention is further illustrated by the following examples which are provided to be exemplary of the invention, and do not limit the scope of the invention.

All chemicals and solvents were of reagent grade and were used without further purification.

### Example 1

The method of obtaining a lipopeptide conjugate *N*^{α}-*lauroyl*-Gly-Pro-Hyp-Lys-NH₂

### a) The method of obtaining Gly-Pro-Hyp-Lys(Boc) loaded aminomethylpolystyrene carrier

The solid support comprises 4-(2',4'-dimethoxyphenyl-Fmoc-aminomethyl)-phenoxyacetamide linker attached to aminomethylpolystyrene (Rink Amide AM (from ChemPep)), with a loading capacity of 0.30÷1.00 mmol/g and particle size: 100÷200 mesh. The aminomethylpolystyrene carrier was swollen in DMF for a period of 1h (15mL·g⁻¹) at 25±2°C and filtrated.

The carrier was preloaded with Fmoc-Lys(Boc)-OH in a two-step procedure. In the first step: a piperidine solution (25% in DMF, 10mL) was added into the reaction vessel and shaken at 25±2°C. After 25 minutes the carrier was filtrated and washed with solvents of DMF, DMF/DCM (1:1, v/v), DCM. In a second step: 0.492g (1.05 mmol) (0.246g (0.52 mmol)) Fmoc-Lys(Boc)-OH (MW=468.54g/mol) and 0.142g (1.05 mmola) (0.071g (0.52 mmola)) HOBt (monohydrate, MW=135.12g/mol) were dissolved in DMF (3mL). Reaction mixture was transfered into reaction vessel before the addition of 162.6µL (1.05 mmol) (81.3µl (0.52 mmol)) DIPCI (MW=126.20g/mol, 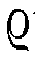 ^{20°C}=0.815g/mL) and shaken for 3 hours at 25±2°C. The carrier was filtrated and washed with solvents of DMF, DMF/DCM (1:1, v/v), DCM.

After the first amino acid was loaded to the polymeric carrier, the unreacted sites had to be endcapped. For this purpose, a fresh solution containing 560.1 µl (5.92 mmol) of acetic anhydride (Ac₂O MW = 102.1 g/mol, 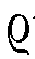 ^{20°C} = 1.08 g/mL) and 318.8 µl (3.95 mmol) of pyridine (MW = 79.1g/mol, 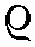 ^{20°C} = 0.98g/mL) was prepared each time in a separate vessel. The mixture was transferred into the vessel with the Fmoc-amino acid preloaded polymeric carrier and was shaken at 25±2°C for 30 minutes, then the carrier was filtrated and washed three times with a series of solvents: DMF, DMF-DCM (1: 1, *ν*/*ν),* DCM, diethyl ether and drying under reduced pressure.

For Fmoc-substituted resin, substitution determination was performed by suspending a 2mg (1,5µmol) sample of dried Fmoc-AK-polymeric support in 3 mL 25% (*ν*/*ν*) piperidine in DMF to generate the dibenzofulvene-piperidine adduct that was quantified by UV-Vis spectroscopy (measuring the absorbance at 298.8 nm wavelength). The resulting Fmoc-AK substitution [mmol/g] reffered to 0.345÷0.790 was calculated with the following formula, L _{Fmoc-AA} = A_{próbki}/(1,75·m_{y}[mg]).

The Fmoc-Lys(Boc)-polymeric support of an Example 1 was used to build peptide chain. The remaining amino acids of the present peptide was added in cyclic two-step fashion. In the first step, Fmoc protecting group was in each cycle removed by adding piperidine solution (25% in DMF) (10mL) into the reaction vessel and shaking at 25±2°C for 1×5min, then 1×15min.

The Fmoc-AK(PG)-OH loaded carrier was filtrated and washed with solvents of DMF, DMF/DCM (1:1, v/v), DCM. Before the coupling step, the peptidyl-carrier was washed again in reverse order. The addition of each Fmoc-AK(PG)-OH was achieved by preparing Fmoc protected amino acid and 0.267 g (1.975 mmol) (0.116g (0.863mmol)) HOBt (monohydrate, MW=135,12g/mol) in 9mL of NMP-DMF-DCM mixture (1:1:1, *ν*/*ν*/*ν*) followed by addition of 305.8µl (1.975mmol) (133.6µl (0.863mmol)) DIPCI (MW=126.20g/mol, 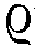 ^{20°C}=0.815g/mL). The reaction vessel was shaken on a laboratory shaker at 25 ± 2°C for a period of 3.5 hours. Coupling reactions with Fmoc-Pro-OH and Fmoc-Gly-OH proceeded at a temperature of 18±2°C for a period of 3.5 hours. Coupling reactions was each time monitored by the chloranil test. Exact amounts of amino acid derivatives: 0.698g (1.975 mmol) (0.305g (0.863 mmol.) Fmoc-Hyp-OH (MW=353.37g/mol), 0.666g (1.975 mmol) (0.291g (0.863 mmol)) Fmoc-Pro-OH (MW=337.37g/mol), 0.587g (1.975 mmol) (0.256g (0.863 mmol)) Fmoc-Gly-OH (MW=297.31g/mol).

### b) The method of obtaining N^{α}-Lauroyl-Gly-Pro-Hyp-Lys(Boc) loaded aminomethylpolystyrene resin

Fragment Gly-Pro-Hyp-Lys(Boc) loaded aminomethylpolystyrene carrier was used to modified with lipid derivative. Two-step procedure includes in the first step adding a piperidine solution (25% in DMF) to the polymeric carrier and shaking the content of the reaction vessel at 25±2°C for 5 minutes and then 15 minutes. In the second step, for the coupling reaction (per 1 g of polymer carrier) 0.395g (1.975 mmol) (0.173g (0.863mmol)) of lauric acid (MW=200.32g/mol, 99,0% purity (Brenntag)) was dissolved in 8mL of DMF with the addition of 1% NSP (99% purity, Sigma Aldrich) and 0.267g (1.975 mmol) (0.116g (0.863mmol)) HOBt (monohydrate, MW=135,12g/mol) and 0.634g (1.975mmol) (0.277g (0.863 mmol)) TBTU (MW=321.09g/mol) were dissolved in 7mL of DMF followed by addition of 688.1µl (3.950 mmol) (300.5µl (1.725 mmola)) of DIPEA (MW=129,25g/mol, 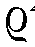 ^{25°C}=0,742g/mL). All the reagents were placed in the reaction vessel each time and shaken on a laboratory shaker at 25 ± 2°C for a period of 3.5 hours. After each deprotection and coupling steps, the peptidyl carrier was washed three times with solvents for 1 min in the following order: DMF, DCM-DMF (1:1, v/v), DCM followed by the chloranil test performed. Before the coupling step, the peptidyl carrier was washed again in reverse order.

### c) The method of obtaining N^{α}-Lauroyl-Gly-Pro-Hyp-Lys-NH₂

The carrier was filtereded through a sintered glass funnel and washed sequentially the carrier twice with DCM, and then with diethyl ether. The dried peptidyl carrier (0.5 g) was placed in a 50 mL round-bottom flask equipped with a magnetic stirrer bar and treated with 20 mL of TFA-PhOH-iPr₃SiH-H₂O (88:5,8:2:4,2, v/v/v/v). The reaction was carried out at 25±2°C for a period of 3h, under nitrogen atmosphere. After the time, the polymer carrier was filtered, washed twice with a small amount of TFA mixture. The filtrate was evaporated to dryness on a rotary evaporator but the residue was dissolved in 10% acetic acid and washed three tmes with diethyl ether. The organic layer was discarded and the aqueous layer was frozen and lyophilized.

The quantity of 220.5 mg of crude product was obtained. The product was purified by loading onto a column packed with the octadecyl groups bounded with silica gel. ((SilicaGel C18 from *Macherey-Nagel,* volume/adsorbent weight 15 mL/2000 mg). Analyte elution was carried out in a solvent gradient with an increasing proportion (8 mL) of solvent B in the range of 0-30% B in A (0.1% TFA in water (A), 80% acetonitrile with 0.1% TFA (B)). Product analysis was based on TLC, HPLC. The purified compound was lyophilized and stored in a vacuum desiccator over the drying agent. After purification, 152.1mg (69.0% yield) of a pale yellow product was obtained with over 98% purity (RP-HPLC). Lipopeptide conjugate (trifluoroacetate salt) with a molecular formula of C₃₀H₅₄N₆O₆+nCF₃COOH+H₂O was characterized by MS, | RP-HPLC. MALDI-TOF-MS; calc. [M+H]⁺, [M+Na]⁺, [M+K]⁺: 595.8, 617.8; 633.8, found, 595.6; 617.5; 633.3, Rf=0.53 (*n*-BuOH-AcOH-H₂O 4:1:2;*ν*/*ν*/*ν*). LC-MS analysis, Rₜ =16.36 [min] (linear gradient in the range of 0÷80% solvent B in A, 30 min (0.1% HCOOH in H₂O (A), 0.1% HCOOH in acetonitrile (B); flow: 0.8ml/min, UV detection at a wavelength of 224÷228nm or 203÷207nm, ESI-MS detection), Column: Eclipse XDB-C8 4.6×150mm (5µm), column temperature: 25°C).

### Example 2

The method of obtaining *N*^{α}-lauroyl-Gly-Hyp-Hyp-Lys-NH₂

### a) The method of obtaining Gly-Hyp-Hyp-Lys(Boc) loaded aminomethylpolystyrene carrier

Fragment loaded aminomethylpolystyrene carrier was synthesized starting from a polymeric carrier preloaded with Fmoc-Lys(Boc)-OH (with 0.345÷0.790 mmol/g loading) according to Example 1a). The peptide fragment was built in the subsequent deprotection/coupling steps. For the coupling reactions, Fmoc-AK-OH and 0.267g (1.975 mmol) (0.116g (0.863 mmol)) HOBt (monohydrate, MW=135.12g/mol) were dissolved in 9 ml of NMP-DMF-DCM (1: 1: 1, *ν*/*ν*/*ν*) followed by addition of 305.8µl (1.975 mmol) (133.6µl (0.863 mmol)) DIPCI (MW=126.20g/mol, 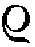 ^{20°C}=0.815g/mL). Exact amounts of amino acid derivatives: 0.698g (1.975 mmol) (0.305g (0.863 mmol) Fmoc-Hyp-OH (MW=353.37g/mol), 0.698g (1.975 mmol) (0.305g (0.863 mmol) Fmoc-Hyp-OH (MW=353.37g/mol), 0.587g (1.975 mmol) (0.256g (0.863 mmol)) Fmoc-Gly-OH (MW=297.31g/mol). All the reagents were placed in the reaction vessel each time and shaken on a laboratory shaker at 25±2°C for a period of 3.5 hours. Coupling reaction with Fmoc-Hyp-OH and Fmoc-Gly-OH derivatives was carried out at 18±2°C for 3.5h.

### b) The method of obtaining N^{α}-Lauroyl-Gly-Hyp-Hyp-Lys(Boc) loaded aminomethylpolystyrene carrier

Fragment Gly-Hyp-Hyp-Lys(Boc) loaded aminomethylpolystyrene carrier (with 0.345 mmol/g loading) was used to prepare lipopeptide conjugate according to the procedure described in Example 5. Subsequent coupling reaction with fatty acid was carried out analogously to Example 1b. In the first step: a piperidine solution (25% in DMF) was added to the carrier and the content of the reaction vessel was shaker at 25±2°C for 5 minutes and then 15 minutes. In the second step, for the coupling reaction (per 1g of polymer carrier) 0.395g (1.975 mmol) (0.173g (0.863mmol)) lauric acid (MW=200.32g/mol, 99,0% purity (Brenntag)) was dissolved in 8mL of DMF with the addition of 1% NSP (99% purity, Sigma Aldrich) and 0.267g (1.975 mmol) (0.116g (0.863mmol)) HOBt (monohydrate, MW=135,12g/mol) and 0.634g (1.975mmol) (0.277g (0.863 mmol)) TBTU (MW=321.09g/mol) were dissolved in 7mL of DMF followed by addition of 688.1µl (3.950 mmol) (300.5µl (1.725 mmola)) DIPEA (MW=129,25g/mol, 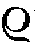 ^{25°C}=0,742g/mL). All the reagents were placed in the reaction vessel each time and shaken on a laboratory shaker at 25 ± 2°C for a period of 3.5 hours.

### c) The method of obtaining N^{α}-Lauroyl-Gly-Hyp-Hyp-Lys-NH₂

Thorough washing and drying must be carried out before cleavage analogusly to Example 1c. The dried peptidyl carrier (0.5 g) was placed in a 50 mL round-bottom flask equipped with a magnetic stirrer bar and treated with 20 mL of TFA-PhOH-iPr₃SiH-H₂O (88:5,8:2:4,2, v/v/v/v). The reaction was carried out at 25±2°C for a period of 3 h, under nitrogen atmosphere.

The quantity of 226.5 mg of crude product was obtained. The product was purified by loading onto a column packed with octadecyl groups boned with silica gel ((SilicaGel C18 from *Macherey-Nagel,* volume/adsorbent weight 15 mL/2000 mg). Analyte elution was carried out in a solvent gradient with an increasing proportion (8 mL) of solvent B in the range of 0―30% B in A (0.1% TFA in water (A), 80% acetonitrile with 0.1% TFA (B)). Product analysis was based on TLC, HPLC. The purified compound was lyophilized and stored in a vacuum desiccator over the drying agent. After purification, 133.6mg (59.0% yield) of a pale yellow product was obtained with over 97% purity (RP-HPLC). Lipopeptide conjugate (trifluoroacetate salt) with a molecular formula of C₃₀H₅₄N₆O₇+nCF₃COOH+H₂O was characterized by MS, RP-HPLC. MALDI-TOF-MS; calc. [M+H]⁺, [M+Na]⁺, [M+K]⁺: 611,8; 633,8; 649,8, found 611,2; 633,2; 649,9, Rf=0.50 (*n*-BuOH-AcOH-H₂O 4:1:2;*ν*/*ν*/*ν*). LC-MS analysis, Rₜ =16,30 [min] (linear gradient in the range of 0÷80% solvent B in A, 30 min (0.1% HCOOH in H₂O (A), 0.1% HCOOH in acetonitrile (B); flow: 0.8ml/min, UV detection at a wavelength of 224÷228nm or 203÷207nm, ESI-MS detection), Column: Eclipse XDB-C8 4.6×150mm (5µm), column temperature: 25°C).

### Example 3

The method of obtaining *N*^{α}-lauroyl-Gly-Pro-Hyp-Lys-OH (lipo-fragment A)

### a) The method of obtaining Gly-Pro-Hyp-Lys(Boc) loaded 2-chlorotrityl chloride carrier

The linear peptide was prepared on 2-chlorotrityl carrier (loading capacity: 1.00÷1.60 g/mmoles, particle size: 100÷200 µm, Novabiochem). The attachment of the first *N*^{α}-Fmoc protected aminoacid proceeded by introdution of 1.181 g (2.52 mmoles) (0.591 g (1.26 mmoles)) of Fmoc-Lys(Boc)-OH (MW=468.54g/mol) dissolved in 10mL of DCM-DMF mixture (9:1, *ν*/*ν*) to the polymeric carrier in addition of 878.1 µl (5.041 mmol) (439.1 µl 2.52 mmol) DIPEA (MW = 129.25 g/mol, 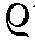 ^{25°C} = 0.742 g/mL). The reaction vessel was shaken at 25±2°C for 120 minutes. The remaining active site of the resin was then capped using 10 mL of fresh mixture of DCM-MeOH-DIPEA (17:2:1, *ν*/*ν*/*ν*). The Fmoc-Lys(Boc)-polymeric carrier was subsequently filtered and washed with DCM (3x), DMF (2x) and DCM (2x), then separated on a sintered-glass filter and dried overnight under vacuum. The loading of the resin (0.655÷1.245mmol/g) was determined spectrophotometrically. The Fmoc-Lys(Boc)-polymeric support of an Example 3 was used to build peptide chain. The remaining amino acids of the present peptide was added in cyclic two-step fashion. In the first step, Fmoc protecting group was in each cycle removed by adding a piperidine solution (25% in DMF) (10mL) into the reaction vessel and shaking at 25±2°C for 1×5min, then 1×15min.

The Fmoc-AK(PG)-OH loaded carrier was filtrated and washed three times with solvents for 1 min in the following order: DMF, DMF/DCM (1:1, v/v), DCM. Before the coupling step, the peptidyl-carrier was washed again in reverse order._The addition of each Fmoc-AK(PG)-OH was achieved by preparing Fmoc protected amino acid and 0.420g (3.113 mmol) (0.221g (1.638 mmol)), HOBt (monohydrate, MW = 135.12 g/mol) in 9mL of NMP-DMF-DCM mixture (1:1:1, *ν*/*ν*/*ν*) followed by addition of 481.9µl (3.113 mmol) (253.6 µl (1.638 mmol)) DIPCI (MW = 126.20 g/mol, 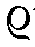 ^{20°C}= 0.815 g/mL). The reaction vessel was shaken on a laboratory shaker at 25±2°C for a period of 3.5 hours. Coupling reactions with Fmoc-Pro-OH and Fmoc-Gly-OH proceeded at a temperature of 18±2°C for a period of 3.5 hours. After each coupling steps, the peptidyl carrier was washed three times with solvents for 1 min in the following order: DMF, DCM-DMF (1:1, v/v), DCM . Coupling reactions was each time monitored by the chloranil test. Exact amounts of amino acid derivatives: Exact amounts of amino acid derivatives: 1.099 g (3.113 mmol) (0.579 g (1.638 mmol) Fmoc-Hyp-OH (MW = 353.37 g/mol), 1.050 g (3.113 mmol) (0.552 g (1.638 mmol)) Fmoc-Pro-OH (MW = 337.37 g/mol), 0.925g (3.113mmol) (0.487g (1.638mmol)) Fmoc-Gly-OH (MW = 297.31 g/mol).

### b) The method of obtaining N^{α}-Lauroyl-Gly-Pro-Hyp-Lys(Boc) loaded 2-chlorotrityl chloride carrier

Fragment Gly-Pro-Hyp-Lys(Boc) loaded 2-chlorotrityl chloride carrier was used to modified with lipid derivative. Deprotection/coupling reactions were carried out at 25 ± 2°C for a period of 3.5 hours (analogously to Examples 1b and 2b).

For the coupling reactions, 0.623 g (3.113 mmol) (0.328 g (1.638 mmol)) of lauric acid (MW = 200.32 g/mol, 99.0% purity (*Brenntag*)), 0.421 g (3.113 mmol) (0.221 g (1.638 mmol)) HOBt (monohydrate, MW = 135.12g / mol), 0.999g (3.113mmol) (0.526g (1.638mmol)) TBTU (MW = 321.09g / mol), 1084.3µl (6.225 mmol) (570.5 µl (3.275 mmol)) DIPEA (MW = 129.25 g / mol, 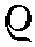 25 ° C = 0.742 g / ml) were used.

### c) The method for preparing of N^{α}-Lauroyl-Gly-Pro-Hyp-Lys-OH

The peptidyl carrier was throughly washed with DCM, diethyl ether and dried in a vacuum desiccator. In the first step: the dried peptidyl carrier (0.5 g) was placed in a 50 mL round-bottom flask equipped with a magnetic stirrer bar and treated with 20 mL of DCM-TFA-iPr3SiH (90:5:5, *ν*/*ν*/*ν*). The reaction was carried out at 25±2°C for a period of 30 minutes, under nitrogen atmosphere. After the time, the polymeric carrier was filtered, washed twice with a small amount of TFA mixture. The filtrate was evaporated to dryness on a rotary evaporator. The second step - the product was treated with 20mL of TFA-DCM (9: 1, v/v) at 5 ° C for a period of 30 minutes, monitoring the reaction by TLC. The reaction mixture was evaporated to dryness and the residue was treated three times with chilled diethyl ether. The product was separated and lyophilized.

The quantity of 355.5 mg of crude product was obtained. The product (250mg/5ml H₂O) was purified by loading onto a column packed with octadecyl groups bonded with silca gel ((SilicaGel C18 from *Macherey-Nagel,* volume/adsorbent weight 15 mL/2000 mg). Analyte elution was carried out in a solvent gradient with an increasing proportion (8 mL) of solvent B in the range of 0-30% B in A (0.1% TFA in water (A), 80% acetonitrile with 0.1% TFA (B)). Product analysis was based on TLC, HPLC. The purified compound was lyophilized and stored in a vacuum desiccator over the drying agent. After purification, 259.6mg (73.0% yield) of a pale yellow product was obtained with over 97% purity (RP-HPLC). Lipopeptide conjugate (trifluoroacetate salt) with a molecular formula of C₃₀H₅₃N₅O₇+nCF₃COOH+H₂O was characterized by MS, RP-HPLC. MALDI-TOF-MS; calc. [M+H]⁺, [M+Na]⁺, [M+K]⁺: 596,8, 618,8; 634,8, found, 596,2; 618,5; 634,7 Rf=0,47 (*n*-BuOH-AcOH-H₂O 4:1:2;*ν*/*ν*/*ν*). LC-MS analysis, Rₜ =15,40 [min] (linear gradient in the range of 0÷80% solvent B in A, 30 min (0.1% HCOOH in H₂O (A), 0.1% HCOOH in acetonitrile (B); flow: 0.8ml/min, UV detection at a wavelength of 224÷228nm or 203÷207nm, ESI-MS detection), Column: Eclipse XDB-C8 4.6×150mm (5µm), column temperature: 25°C).

### Example 4

The method of obtaining *N*^{α}-lauroyl-Gly-Pro-Lys-OH (lipo-fragment A)

### a) The method of obtaining Gly-Pro-Lys(Boc) loaded 2-chlorotrityl chloride carrier

Preloaded Fmoc-Lys(Boc)-polymer carrier with 0.655÷1.245 mmol/g loading was used. For the coupling reactions, Fmoc-AK-OH and 0.420g (3.113 mmol) (0.221g (1.638 mmol)), HOBt (monohydrate, MW = 135.12 g/mol) were dissolved in 9 ml of NMP-DMF-DCM (1: 1: 1, *ν*/*ν*/*ν*) followed by addition of 481.9 µl (3.113 mmol) (253.6 µl (1.638 mmol)) DIPCI (MW = 126.20 g/mol, 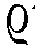 ^{20°C} = 0.815g/mL). Exact amounts of amino acid derivatives: 1.050g (3.113mmol) (0.552g (1.638mmol)) Fmoc-Pro-OH (MW = 337.37g/mol), 0.925g (3.113mmol) (0.487g (1.638mmol)) Fmoc-Gly-OH (MW = 297.31 g/mol). All the reagents were placed in the reaction vessel each time and shaken on a laboratory shaker at 25 ± 2°C for a period of 3.5 hours.

### b) The method of obtaining N^{α}-Lauroyl-Gly-Pro-Lys(Boc) loaded 2-chlorotrityl chloride carrier

Fragment Gly-Pro-Lys(Boc) loaded 2-chlorotrityl chloride carrier was used to modified with lipid derivative. Coupling reaction with fatty acid was carried out at 25 ± 2°C for a period of 3.5 hours (analogously to Example 3b). For the coupling reaction, 0.623 g (3.113 mmol) (0.328 g (1.638 mmol)) of lauric acid (MW = 200.32 g / mol, 99.0% purity (Brenntag)), 0.421 g (3.113 mmol) (0.221 g (1.638 mmol) ) HOBt (monohydrate, MW = 135.12g / mol), 0.999g (3.113mmol) (0.526g (1.638mmol)) TBTU (MW = 321.09g / mol), 1084.3µl (6.225 mmol) (570.5 µl (3.275 mmol)) DIPEA (MW = 129.25 g / mol, 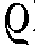 25 ° C = 0.742 g / ml) were used.

### c) The method of obtaining N^{α}-Lauroyl-Gly-Pro-Lys-OH

Clevage and deprotection procedures were carried out according to the Example 3c. In the first step - the dried peptidyl carrier (0.5 g) was placed into a 20 mL round flask equipped with a magnetic stirrer bar and treated with 20mL of DCM-TFA-iPr₃SiH (90: 5: 5, v / v / v) at a temperature of 25 ± 2 ° C for a period of 30 minutes, under nitrogen atmosphere. The filtrate was evaporated to dryness on a rotary evaporator. The second step - the product was treated with 20mL of TFA-DCM (9: 1, v/v) at 5 ° C for a period of 30 minutes, then the reaction mixture was evaporated to dryness and the residue was treated three times with chilled diethyl ether. The product was separated and lyophilized. The quantity of 285.4 mg of crude product was obtained. The product (150mg/5ml H₂O) was purified by loading onto a column packed with octadecyl groups Bondem with silica gel ((SilicaGel C18 from *Macherey-Nagel,* volume/adsorbent weight 15 mL/2000 mg). Analyte elution was carried out in a solvent gradient with an increasing proportion (8 mL) of solvent B in the range of 0-30% B in A (0.1% TFA in water (A), 80% acetonitrile with 0.1% TFA (B)). The purified compound was lyophilized and stored in a vacuum desiccator over the drying agent. After purification, 180.3mg (63.0% yield) of a pale yellow product was obtained with over 96% purity (RP-HPLC). Lipopeptide conjugate (trifluoroacetate salt) with a molecular formula of C₂₅H₄₆N₄O₅+nCF₃COOH+H₂O was characterized by MS, RP-HPLC. MALDI-TOF-MS; calc. [M+H]⁺, [M+Na]⁺, [M+K]⁺: 483.7; 505.7; 521.7, found 483.3; 505.7; 521.4, Rf=0,41 (*n*-BuOH-AcOH-H₂O 4:1:2;*ν*/*ν*/*ν*). LC-MS analysis, Rₜ =15,48 [min] (linear gradient in the range of 0÷80% solvent B in A, 30 min (0.1% HCOOH in H₂O (A), 0.1% HCOOH in acetonitrile (B); flow: 0.8ml/min, UV detection at a wavelength of 224÷228nm or 203÷207nm, ESI-MS detection), Column: Eclipse XDB-C8 4.6×150mm (5µm), column temperature: 25°C).

### Example 5

The method for polycondensation reaction of the peptide fragments exemplified by the preparation of *N*^{α}-*lauroyl*-Gly-Pro-Hyp-Lys-Gly-Hyp-Hyp-Lys-NH₂

### I. The method of obtaining a Fragment B (Gly-Hyp-Hyp-Lys(Boc)) loaded aminomethylpolystyrene carrier

Fragment B loaded aminomethylpolystyrene carrier was synthesized starting from a polymeric carrier preloaded with Fmoc-Lys(Boc)-OH (0.345mmol/g, scale: 0.345 mmol). Every subsequent Fmoc deprotection step was performed with a piperidine solution (25% in DMF) at 25±2°C for 5 min and then 15 min. Coupling reactions were performed using a 2.5-fold excess of activated in situ Fmoc-AK-OH, 133.5 µl (0.863 mmol)) DIPCI and 0.116 g (0.863 mmol) HOBt (monohydrate, MW = 135.12 g/mol) in a solvent mixture of NMP-DMF-DCM (1: 1: 1, v/v/v) (9mL). For the coupling reactions, 0.305 g (0.863 mmol) Fmoc-Hyp-OH (MW = 353.37 g/mol), 0.305 g (0.863 mmol) Fmoc-Hyp-OH (MW = 353.37 g/mol), 0.256 g (0.863 mmol) of Fmoc-Gly-OH (MW = 297.31 g/mol) were used. Before acid cleavage, Fmoc protected peptide-polymer support was treated with a 25% solution of piperidine in DMF at room temperature for 5 min, 15 min. The polymeric carrier was removed by filtration under reduced pressure, washed twice with DCM, diethyl ether and dried. Fragment B was characterized upon treatment of polymer support sample with a mixture of TFA according to the procedure described in Example 2c. The final product Gly-Hyp-Hyp-Lys-NH₂ was identified by mass spectrometry. MALDI-TOF-MS; calcd [M+H]⁺, [M+Na]⁺, [M+K]⁺: 429.5; 451.5; 467.5 found 429.2; 451.1; 467.6.

### II. The method of obtaining N^{α}-lauroyl-Gly-Pro-Hyp-Lys(Boc)-OH (lipo-fragment A)

Lipo-fragment A supported on 2-chlorotrityl carrier was prepared according to the protocol (loading of 0.655 mmol/g, scale: 1.310 mmol). Lipo-fragment A release was accomplished by placing the dried peptidyl-carrier in a round bottom flask 50mL equipped with a magnetic stirring bar and adding 20mL of the DCM-TFA mixture (95:5, *ν*/*ν*). The reaction was carried out for 30 minutes under nitrogen atmosphere, then polimeric carrier was filtered, washed twice with small amount of TFA mixture (with a mixture of TFA (2x2mL)). The filtrate was concentrated and treated with chilled diethyl ether. The solvent was decanted each time and poured with a fresh portion. The resulting protected peptide (lipo-fragment A) was separated, dissolved in water, neutralized with 0.01M KOH and then extracted three times with DCM. The combined organic extracts were dried and evaporated. The product was confirmed by mass spectra. MALDI-TOF calculated [M+H]⁺; [M+Na]⁺; [M+K]⁺: 696.9; 718.9; 734.9, found 696.3; 718.2; 734.7. The product without additional steps was isolated in a sufficient purity (>95%).

### III. Polycondensation reaction of the peptide fragments: lipo-fragment A and fragment B to receive N^{α}-lauroyl-Gly-Pro-Hyp-Lys-Gly-Hyp-Hyp-Lys-NH₂

0.481 g (0.692 mmol) of lipo-fragment A was dissolved in 8 mL of DMF, 0.444 g (1.384 mmol) of TBTU (MW = 321.09 g/mol) and 0.187 g (1.384 mmol) of HOBt monohydrate (MW = 135.12 g/mol) in 10ml DMF were added. 482.2 µl (2.768 mmol) of DIPEA (MW = 129.25 g/mol, 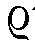 ^{20°C}= 0.742 g/mL) was added into the mixture. The whole mixture was transferred into a reaction vessel with 0.5 g of peptidyl carrier (Fragment B) (scale: 0.173 mmol) and shaken at 25±2°C for a period of 2.5÷5h. Coupling completeness was monitored by the chloranil test. The polymeric carrier was filtered, washed successively with DMF, DCM, diethyl ether, dried. Lipopeptide conjugate release was accomplished by placing the dried peptidyl-carrier in a round bottom flask 50mL equipped with a magnetic stirring bar and adding 20mL of the mixture TFA-PhOH-iPr₃SiH-H₂O (88:5,8:2:4,2, *ν*/*ν*/*ν*/*ν*).

The reaction was carried out at 25±2°C for a period of 3 h under a nitrogen atmosphere. After the time, the polymer support was filtered, washed twice with a small amount of TFA mixture. The filtrate was evaporated to dryness. The residue was dissolved in 10% acetic acid and washed three times with diethyl ether. The organic layer was discarded and the aqueous layer was frozen and lyophilized. The quantity of 148.5 mg of crude product was obtained. The product was identified by RP-HPLC (Column: Eclipse XDB-C8 4.6×150mm (5µm), t_{R} = 16.56, linear gradient in the range of 0÷80% solvent B in A, 30 min (0.1% HCOOH in H₂O (A), 0.1% HCOOH in acetonitrile (B); flow: 0.8ml/min, UV detection at a wavelength of 224÷228nm or 203÷207nm, ESI-MS detection) and MALDI-TOF-MS calcd [M+H]⁺: 1007.2, found 1007.5. The product was purified by loading onto a column filled with silica gel modified with octadecyl groups ((SilicaGel C18 from *Macherey-Nagel,* volume/adsorbent weight 15 mL/2000 mg). Analyte elution was carried out in a solvent gradient with an increasing proportion (8 mL) of solvent B in the range of 0-50% B in A (0.1% TFA in water (A), 100% acetonitrile with 0.1% TFA (B)). The purified compound was lyophilized and stored in a vacuum desiccator over the drying agent. After purification, 111.4mg (75.0% yield) of a pale yellow product was obtained with over 95% purity (RP-HPLC).

### Example 6 Optimization of reaction conditions for obtaining Hyp-rich lipopeptide conjugates

### a) Exploring the coupling reaction between the free α-amino group of the amino acid residue in the sequence of L-4-hydroxyproline-containing peptidyl carrier and the carboxyl group of the successively coupled Fmoc-protected amino acid residue to build hydroxyproline-rich lipopeptide conjugates

**Table 2**

| **Methods** | **Reaction condition for peptide bond formation** | | | | | **Yield [%]¹** |
|---|---|---|---|---|---|---|
| | **Coupling reagents** | **Solvents** | **Molar ratio of used reagents** | **Reaction temperature [°C]** | **Reaction time [h]** | |
| **1** | **DIPCl/HOBt** | **NMP/CH₂Cl₂/DMF (1:1:1,*ν*/*ν*/*ν*)** | **Fmoc-AA-OH/DIPCI/HOBt (2:2:2)** | **18** | **3-6** | **60-69** |
| **2** | **DIPCI/HOBt** | **CH₂Cl₂/DMF (1:1, *ν*/*ν*)** | **Fmoc-AA-OH/DIPCI/HOBt (1:1:1)** | **25** | **4-8** | **46-50** |

| | | | | | | |
|---|---|---|---|---|---|---|
| **¹Percent yield of the compound after purification** | | | | | | |

### b) Evaluation of scale-up process for synthesis of lipopeptide conjugates

The lab scale-up of lipopeptide conjugates used in biological system was performed following the same protocol. The method was efficient for preparing lipopeptide conjugates with a safety profile. The reproducibility (n=3) was estimated increasing the scale of process (from 0.395 mmol to 1.185 mmol) - Table 3. Efficiency measurements of scaling process was based on limit parameters of the yield (59%±10%) and degree of purification >97%±3%.

**Table 3**

| **The scale of a process [mmol]** | **The volume of reaction vessel [mL]** | **Aminomethylpolystyrene carrier [g]** | **Yield [%]¹** | **Purity[%]** |
|---|---|---|---|---|
| **0.395** | **6-14** | **0.5** | **59** | **99²** |
| **0.790** | **25-50** | **1.0** | **65** | **97²** |
| **1.185** | **50-100** | **1.5** | **60** | **95²** |

| | | | | |
|---|---|---|---|---|
| **¹Percent yield of the compound after purification; ²Purity determination was achieved by RP-HPLC analysis** | | | | |

Pharmaceutically acceptable salts of lipopeptide conjugates were obtained *in situ* final isolation and purification according to the invention or by reacting the free basic group with the appropriate organic acid (trifluoroacetate, acetate) or mineral acid (hydrochloride, hydrobromide, hydroiodide, hydrofluoride).

### Examples of Applications

### Example 7

Assessment of cell migration and reduction of wound surface area in response to lipopeptide conjugate therapy

*In vitro* wound healing (closure) assay using IBIDI culture inserts were performed according to literature. Briefly, cell suspension (1,5×10⁴) was seeded in Culture-insert 2 well in 70µl of growth medium (DMEM) and cultured for 24 h (37°C, 5%CO₂) to form a monolayer. After removal of the Culture-insert, the newly created cell-free gap (wound) was wahed with DMEM followed by PBS. Cell monolayer was also preincubated for 30 min in the presence of mitomycin C (8µg/mL), as a DNA replication and cell proliferation inhibitor. A fresh DMEM and the cells were treated with a pharmaceutical composition containing a lipopeptide conjugate or pharmaceutically acceptable salt and incubated for 24h (37°C, 5%CO₂). The cultures in DMEM (K+) was incubated under the same conditions as described above. After overnight incubation, the media was removed, cells were rinsed with sterile PBS and then 1% paraformaldehyde (PFA) was added. After 20 minutes, the cells were washed with PBS and stained with 0.05% crystal violet solution. The stained cells were washed with PBS and then distilled water was added to each well. The wounded areas were visualized microscopically and photographed by microscope (Zeiss). Photographs from the process of human fibroblasts migration after treatment with a pharmaceutical composition containing a lipopeptide conjugate or a pharmaceutically acceptable salt thereof at a given concentration are shown in Figure 7. Cell migration was analyzed using Imaging Software NIS-Elements Basic Research. Alternatively, the migration rate was expressed as the percentage of area reduction (wound closure) compared to the control K. Data of at least independent experiments (n=3) were shown in Figure 4. The results of the assay indicated that lipopeptide conjugates treatment promotes cell migration, contributing to promote the wound healing process. However, lipopeptide conjugates at a concentration of 0.01µg/mL regulated independently both processes, including fibroblast proliferation and migration.

The wound area reduced by 10.3-20.7% as cell migration progresses in comparison to control. This effect was statistically significant. The migration of primary fibroblast cells was enhanced after 24 hours of lipopeptide conjugates treatment. However, the effect of lipopeptide conjugate on migratory capacity of these cells was concentration dependent. Percentage wound area reduction was observed with the use of pharmaceutical composition examples comprising lipopeptide conjugate 1 or 2 in 10-fold increased concentrations. Lipopeptide conjugate 3 facilitates primary human fibroblast cell migration in a lower concentration (<0.1 µg/mL). A reduction of the wound surface by 18.29% compared to the control was achieved with aqueous solution of lipopeptide conjugate at a concentration of 0.01 µg/mL. The results showed that a pharmaceutical composition containing lipopeptide conjugates has also a positive effect on re-epithelization by especially promoting keratinocyte proliferation (Table 4).

Physiologically healed wound area under physiological conditions (K control) was defined as 100%. Wound surfaces after treatment with the pharmaceutical composition containing the lipopeptide conjugate is shown with reference to control K. *In vitro* proliferative activity of cells in the wound environment stimulated with pharmaceutical composition (aqueous solution) containing lipopeptide conjugate at concentrations of 0.01 µg/mL and 0.1 µg/mL are shown as n-fold increase relative to culture in non-stimulated DMEM medium (K, control). Cell proliferative activity of skin cells cultured in the presence of FCS(K+, positive control).

**Table 4**

| **Lipopeptide conjugate** | **Wound closure** | | | | |
|---|---|---|---|---|---|
| | **Keratinocyte proliferation [n-times of control]¹** | | | **Keratinocyte migration [% control]²** | |
| **Concentration [µg/mL]** | **0.01** | **0.10** | **FCS** | **0.01** | **0.10** |
| **1** | **1.08±0.04** | **1.08±0.04** | **1.37±0.12** | **98.70 (94.10÷ 115.40)** | **103.27 (96.00÷106.30)** |
| **2** | **1.33±0.22** | **1.42±0.26** | **1.66±0.30** | **100.40 (96.80÷ 110.00)** | **106.93 (102.50÷114.20)*** |
| **3** | **1.29±0.17** | **1.34±0.11** | **1.38±0.16** | **107.40 (100.20+110.90)** | **102.21 (98.60÷108.00)** |

| | | | | | |
|---|---|---|---|---|---|
| **Keratinocyte proliferation and migration was assessed according to described procedure.** **¹Results of in *vitro* proliferative activity of cells stimulated with pharmaceutical compositions containg lipopeptide conjugates are shown as n-fold increase relative to culture in non-stimulated DMEM medium (K, control).** **The results were presented as mean±SD. Data are representative of three independent experiments. Statistical differences in relations to control, *p*<0.05. ²Results present migration capacity of cells, wound surfaces after treatment with the pharmaceutical composition containing lipopeptide conjugates with reference to control K. Results represent median (min-max) from 2/3 cycle of experiments. Statistical differences in relations to control, *p*<0.05** | | | | | |

### Example 8

### Assessment of skin cell proliferation after stimulation with lipopeptide conjugates

Biological activity of pharmaceutical compositions containing lipopeptide conjugates or their pharmaceutically acceptable salts was determined by skin cells proliferation measurement using an XTT (2,3-bis(2-methoxy-4-nitro-5-sulfophenyl)-2H-tetrazole-5-carboxanilide sodium salt)). The XTT cell proliferation assay was carried out according to protocols previously reported by Barabowska―Rybak et al. (2013), Kukowska et al. (2016). Approximately, 5×10³ fibroblast human cell line BR1N, human primary fibroblast cells or HaCaT cells in 200 µl DMEM supplemented with 10% FCS per well were seeded in 96-well plate and incubated for 24 h (37°C, 5%CO₂).

The cells were removed from each well by rinsing DMEM. The fresh DMEM and cells were exposed to lipopeptide conjugate or its pharmaceutically acceptable salt solutions (pharmaceutical composition) at the final concentrations of 0.001, 0.01, 0.1, 1.0, 10.0, 25.0 µg mL⁻¹ and incubated for 48 hours at 37°C (5%CO₂). Cells in DMEM supplemented with 10% FCS (K+) and in DMEM (K) were incubated under the same conditions as described above.

The number of viable cells was assessed after the time of incubation by adding XTT solution. The estimation was based on the formation of a colored formazan product in the presence of intact metabolic acivity in living cells. The absorbance of soluble formazan product was measured in cellular supernatants spectrophotometrically at a wavelenght 490nm. The results are the average of 3 independent cycles of experiments, they represent the relative number of cells treated with a pharmaceutical composition containing lipoeptide conjugates or its pharmaceutically acceptable salts at specific concentrations (Figure 5, Figure 6, Table 3). Cells in DMEM medium after the period of incubation provide a 100% relative numerous cells (K).

Lipoeptide conjugates provide an optimal environment for the growth, development and viability of skin cells. The cellular biological responses (cellular growth and proliferation) was observed after exposure to pharmaceutical composition containing lipopeptide conjugate or its pharmaceutically acceptable salt at concentrations. Significant increases in cell proliferation was observed after exposure to pharmaceutical composition containing lipopeptide conjugate or its pharmaceutically acceptable salt at concentrations of 0.01µg/mL i 0.1µg/mL. The biological effect (increase proliferation activity of keratinocytes as well as fibroblasts) was noticed at wide range of concentrations of lipopeptide conjugates. The strongest proliferative effect on keratinocytes was comparable to the effect induced by the mitogenic factor, FCS (p<0.05).

### Example 9

### Cytotoxicity assessment of lipopeptide conjugates (safety assessment)

The cytotoxicity of lipopeptide conjugates or their pharmaceutically acceptable salts was assessed in immortalized human cell lines, human primary fibroblast cells independently, separately using two assays, XTT and LDH. Approximately, 5×10³ fibroblast human cell line BR1N, human primary fibroblast cells or HaCaT cells in 200 µl DMEM supplemented with 10% FCS per well were seeded in 96-well plate and incubated for 24 h (37°C, 5%CO₂). Culture medium was replaced with fresh DMEM, and then the cells were exposed to lipopeptide conjugate or its pharmaceutically acceptable salt solutions administered in DMEM at the final concentration in the range from 1 *µ*g mL⁻¹ to 1000 *µ*g mL⁻¹. In the experiments, cells in DMEM medium treated with 0.9% solution of Triton X-100 were used as positive control (K+, 100% mortality), untreated cells in DMEM medium were assayed as a negative control (K-). After incubation, cytotoxicity was assessed by measuring the activity of the intracellular cytoplasmic enzyme - lactate dehydrogenase (LDH) and decrease in cell viability measured by a decrease in mitochondria metabolism activity to XTT reduction. The rapidly release of LDH enzyme into the cell culture supernatant is correlated with the plasma membrane damage and cell death. The LDH enzyme release was measured after removal of the supernatant, colorimetrically by incubation with reagent containing 2[4-iodophenyl]-3-[4-nitrophenyl]-5-phenyltetrazolium chloride. The absorbance measured at a wavelenght of 490nm using microplate reader Perkin Elmer spectrophotometer was proportional do LDH activity and the number of damaged cells. The XTT test was performed by adding the XTT solution to each test well. After incubation time at 37°C, the absorbance was measured at a wavelenght of 490nm. Average of absorbance measurements from 3 independent experiments was used to estimate the cell death. The mean absorbance from control wells was considered as 0% (negative control) and 100% (positive control) of cell mortality.

Results based on both LDH, XTT assay (Table 5) suggest especially safety profile of lipopeptide conjugates and their tolerance for skin cells. No cytotoxic effect on skin cells was observed at concentration well above biological effect. Lipopeptide conjugates exerted a cytotoxic effect at the higher milimolar concentrations (10⁻⁴ M). Increase in LDH activity correlated with decrease of skin cell viability was observed after the use of lipopeptide conjugates above concentration of 1,71 · 10⁻⁴M (p<0,05 vs. control).

Effective concentrations (EC₁₀) causing 10% loss of cell viability were shown in Table 2. Effective concentration (EC₅₀) causing 50% loss of cell viability was observed to human primary fibroblast cells at concentration above 6,85 · 10⁻⁴M. The effect assessed using XTT test was observed at lower concentration and preceded the cell membrane damage leading to LDH release. EC₅₀ values estimated by LDH assay was above the concentration 1,13 · 10⁻³M.

**Statistical data analysis**. All data obtained from meaasurements were analyzed using Statistica ver. 8.0 and Microsoft Excel ver. 2010. The studies compared several variable parametric ANOVA test was used in combination with *post hoc* test NIR (p<0.05 were considered statistically significant).

**Table 5**

| **Test** | **Cell lines** | **Lipopepide conjugate 1** | | **Lipopepide conjugate 2** | | **Lipopepide conjugate 3** | |
|---|---|---|---|---|---|---|---|
| | | **EC[µM]** | | | | | |
| | | **EC₁₀** | **EC₅₀** | **EC₁₀** | **EC₅₀** | **EC₁₀** | **EC₅₀** |
| **LDH** | **HaCaT** | **537.1±45.3** | **nd** | **346.9±35.0** | **nd** | **171.4±78.3** | **nd** |
| | **primary fibroblast** | **890.9±171. 9** | **nd** | **409.6±66.9** | **1131.7±337 .5** | **420.6±42.1** | **1682.3±420.6** |
| | **BR1N** | **nd** | **nd** | **432.8±34.5** | **nd** | **nd** | **nd** |
| **MTT** | **HaCaT** | **254.1±85.5** | **nd** | **nd** | **nd** | **210.2±110.4** | **nd** |
| | **primary fibroblast** | **722.2±177. 2** | **nd** | **431.2±34.7** | **819.1±163. 8** | **399.7±35.8** | **685.0±137.5** |
| | **BR1N** | **nd** | **nd** | **327.6±40.9** | **nd** | **>504.7** | **nd** |

| | | | | | | | |
|---|---|---|---|---|---|---|---|
| nd - not determined at tested concentration range of lipopeptide conjugate;¹ 1÷1000µg/mL | | | | | | | |

### Example 10

### Assessment of hemolytic activity of lipopeptide conjugates (safety assessment)

Hemolytic activity was determined by incubating 2% suspension of erythrocytes from healthy human blood (n=3)) with serial dilutions of each selected lipopeptide conjugates (at concentration range from 1000µg/mL to 10µg/mL). Fresh erythrocytes were collected by centrifugation for 10 min at 500xg, washed three times with PBS and diluted to a concentration of 2% in PBS. Erythrocytes were incubated with lipopeptide conjugate, but also with 1% Triton X-100 (positive control), in PBS (blank) at 37°C for 1 hour. After incubation, the plates were centrifuged at 500×g for 10 min, and the supernatants were transferred to new clean wells. Release of hemoglobin was measured as the absorbance of the supernatants at a wavelength of 540 nm using microplate reader (Victor PerkinElmer). The relative optical density compared to the suspension treated with 1% Triton X-100 was defined as the percentage of hemolysis. The results present HC₁₀, HC₅₀ defined as the lowest concentration of lipopeptide conjugate that induced 10% and 50% cell lysis, respectively. The novel lipopeptide conjuagtes have a lack of hemolytic activity at the wide range of concentrations in relation to their biological effect. These lipopeptide conjugates induced 10% of cell lysis at concentration range from 0.19 to 1.62 mM (Table 6). No strong hemolytic effect inducing 50% of cell lysis was observed at the whole range of concentration.

**Table 6**

| **Example of lipopeptide conjugates** | **Hemolytic activity** | |
|---|---|---|
| | **HC₁₀ [µg/mL]** | **HC₅₀ [µg/mL]** |
| **1** | **142.5 (0.2962 mM)** | **>500 (1.0387 mM)** |
| **2** | **113.4 (0.1858 mM)** | **nd** |
| **3** | **961.1 (1.6169 mM)** | **nd** |

| | | |
|---|---|---|
| **HC₁₀; HC₁₀ defined as the lowest concentration of lipopeptide conjugate that induced 10% and 50% cell lysis, median, 25÷75%; nd - 50% of cell lysis was not determined after application of lipopeptide conjugate, ^{∗}10÷500µg/mL** | | |

### Example 11

### Hydrogel wound dressings FA₁÷FA₄ preparation

Carboxymethylcellulose sodium salt (NaCMC C5678, Sigma Aldrich, viscosity 50÷200cP for 4% aqueous solution at 25°C) was added in portions into phosphate buffer solution (0.05M, pH 7.2) and mixed gently to obtain the desired degree of dispersion followed by sterilization. Preparation of the wound dressing required dropping a pharmaceutical composition or preparation comprising a lipopeptide conjugate or its pharmaceutically acceptable salt to NaCMC at 25°C. A properly prepared hydrogel was stored at 1÷5°C.

**Table 7 The composition of hydrogels (hydrogel wound dressings)**

| Hydrogel wound dressing Components % (w/w) | **FA₁** | **FA₂** | **FA₃** | **FA₄** |
|---|---|---|---|---|
| CMC (carboxymethylcellulose sodium salt) | 8.5 | 8.5 | 8.5 | 8.5 |
| Phosphate buffer solution | 87.5 | 87.5 | 87.5 | 87.5 |
| Lipopeptide conjugate 1 | 2.0 | 0 | 0 | 0 |
| Lipopeptide conjugate 2 | 0 | 2.0 | 0 | 0 |
| Lipopeptide conjugate 3 | 0 | 0 | 2.0 | 0 |
| Distilled water | to 100 | to 100 | to 100 | to 100 |
| Preservative | 0 | 0 | 0 | 0 |

### Example 12

### Preparation of exemplary emulsions FB i FC

All ingredients of the water and oil phases of the emulsion were heated in separate vessels to a temperature of 75°C. The two phases were combined together with further mixing to achieve homogenization. After cooling to room temperature, the lipopeptide conjugate or its pharmaceutically acceptable salt and preservative were added, then the pH was adjusted (5 ÷ 7).

**Table 8. Composition of oil-in-water (o/w) emulsion spray (FB) of U.S. Pat. No. US20050255048, comprising lipopeptide conjugates**

| **Oil phase ingredients** | **Water phase ingredients** | **Other ingredients** |
|---|---|---|
| Polyglyceryl-3-methylglucose distearate 1.5% | Deionized Water to 100% | Lipopeptide conjugate 2% |
| Mineral oil 2.75% | Polypropylene glycol 2,5% | Propellant HFC 134a (1,1,1,2-tetrafluoroethane) 50%* |
| Glyceryl Stearate 0.9% | Glycerine 1,25% | Preservative: Methylisothiazolinone, Methyl-4-hydroxybenzoate, Ethyl-4-hydroxybenzoate (Microcare MEM, Thor Personal Care) 0,8% |
| Stearyl Alcohol 0.4% | | |

| | | |
|---|---|---|
| *70% concentrate, 30% HFC, 3g propellant na 7g concentrate | | |

**Table 9 Composition of oil-in-water (o/w) emulsion (FC) comprising lipopeptide conjugates**

| **Oil phase ingredients** | **Water phase ingredients** | **Other ingredients** |
|---|---|---|
| Polyglyceryl-3-methylglucose distearate 3% | Deionized Water to 100% | Lipopeptide conjugate 2% |
| Glyceryl Stearate 2% | Polypropylene/Polybutylene glycol 1.5% | Preservative: Methylisothiazolinone, Methyl-4-hydroxybenzoate, Ethyl-4-hydroxybenzoate (Microcare MEM, Thor Personal Care) 0.8% |
| Stearyl Alcohol 1% | Glycerine 2.5% | |
| C12-15 Alkyl Benzoate 9.5% | | |
| Caprylic/Capric Acid Triglyceride 9.5% | | |

### Example 13

### In vivo evaluation of the wound healing activity of lipopeptide conjugates

8-10 week-old female *Mus musculus* BALB/c mice (purchased from Tri-City Academic Laboratory Animal Centre-Research nad Services Centre) were used in the study. The experiments were carried out in Tri-City Academic Laboratory Animal Centre - Research and Services Centre according to the approved guidelines of Local Ethics Committee of Animal Experimentation in Bydgoszcz (permit No. 31/2018). Mice were housed under standard conditions, with *ad libitum* access to food pellets and tap water, and kept under control conditions (22±2°C, relative humidity 55±10%, 12/12 light/darck cycle).

In experiments, mice were divided into groups i.e. control groups (n=6) and experimental groups (n=6). The animals were anesthetized with 3÷5% isoflurane. After the back of mice was shaved, disinfected, the skin was placed in a fold running paralel to the spine, which was then punctured with a sterile 6mm diameter (biopsy) punch the formation of two symmetrical wounds. Six animals (in the experimental group) were treated with a preparation or a hydrogel wound dressing (hydrogel) containing lipopeptide conjugates or their pharmaceutically acceptable salts. Then, they were covered with a transparent and semi-permeable Tegaderm^{®} dressing (3M, Trans Med. Medical) with dimensions of 2x3cm and secured. The degree of wound closure over time was compared in the group of control animals, i.e. gel control (FA₄) was used. A system of application of a preparation or a wound dressing containing lipopeptide conjugates or their pharmaceutically acceptable salts was developed (the time of staying in animal's wound, taking into account the process of free release from polimer matrix), from daily applications to several days, with the replacement each time during the time of treatment. The progress of the wound healing process was assessed according to the defined administration schedule at intervals (d0, d3, d7, d10, d14). After removal of a preparation on the last day of experiment, the animals were sacrificed and fragments of the restored skin along with a 0.5cm fragment surrounding were collected, frozen and stored. Wound surfaces were each photographed with a Nikon Coolpix S5100 camera and calculated with the use of Image J. Wound healing (restoration of a full-layer skin) is interpreted as the percentage reduction of the original wound area over time. The process of reepithelization was also assessed, indicating the surface of new epithelium extending. The epithelization process is estimated as the difference in wound area reduced in time to wound area (baseline).

### Example 14

### In vivo diabetic wound healing effect of lipopeptide conjugates

In these experiments, we investigated how the lipopeptide conjugates or their pharmaceutically acceptable salts treatment affect full-thickness skin wound healing in the genetically diabetic (db/db) mouse model of type 2 diabates mellitus. Diabetic manifestation in leptin receptor-deficient db/db mice includes obesity, hyperglycemia, hyperinsulinemia, impaired wound-healing. 12-week-old female mice *Mus musculus db*/*db* BKS.Cg-Dock+/+Lepr/J used in this study were obtained from Charles Rivers. The experiments were carried out in Tri-City Academic Laboratory Animal Centre - Research and Services Centre according to the approved guidelines of Local Ethics Committee of Animal Experimentation in Bydgoszcz (permit No. 31/2018). Mice were housed under standard and control conditions before start of the study (at diabetic state develop). The animals were weighed and measured for blood glucose with the One Touch^{®} Blood Glucose Meter (LifeScan Inc., Milpitas, CA). At the beginning of experiments the blood glucose levels in experimental groups remained at the level of 27,39±4,6mmol/l (FA₁), 28,90±1,08mmol/l (FA₂),22,69±5,97mmol/l (FA₃), in control group was 26,30±3,22mmol/l (FA₄). The way of application of a preparation manufactured according to present invention was the same as described in Example 13. In each experiments, groups of six mice were topically treated with sterille preparations FA₁-FA₃, group of six mice (in control) was treated with a sterille hydrogel (FA₄). A transparent dressing (Teagderm^{®}) was applied to cover the wounds after topical application of each preparations. The wound-healing kinetics were observed over a period of 18-day treatments of such preparations. During the experiments, the animals health and weight was monitored. At the beginning of experiments the weight of mice ranged from 51.5g to 70.6g. The mean body weight of the mice in control group was 62.0 g. The mean body weight loss over time was in the control group calculated as 14,61±6,28g (FA₄) and in experimental groups was 14,72±3,46g (FA₁), 15,56±5,03g (FA₂), 9,68±2,77g (FA₃). The differences in body weight loss between two groups were not statistically significant. The mean blood glucose level during the experiments in each groups was calculated as 22,99±5,72mmol/l (FA₁), 27,39±3,16mmol/l (FA₂), 19,87±6,49mmol/l (FA₃), control group: 23,53±4,26mmol/l (FA₄).

Sections from formalin-fixed and parafin-embedded tissues from non-diabetic (n=3) and diabetic mice (experimental groups (n=3), and control group (n=3)) were obtained and used to histopathological study (by using H&E, Masson's trichrome staining). This study show that treatment with lipopeptides or their pharmaceutically acceptable salts positively impacts components of the wound healing process, including restoration of full-thickness soft tissue defects. The effects of the treatment on the wound-healing activity levels in mice with excision wounds was noticed from Day 3. In this model, mice wounds treated with lipopeptde conjugates or their pharmaceutically acceptable salts exhibited the progressive reepithelization (coverage with a new epithelial layer visible) from Day 3, compared to the control group treated with a hydrogel dressing (FA₄) as a base of new/next generation medical products available for wounds. The present study demonstrated accelerated closure of skin wounds in mice from day 7 to 10. The wound healing activity levels (reduction of wound areas and new epithelium coverage) in mice treated with preparations containing lipopeptide conjugates in a therapeutically effective amount were significantly rised on Day 10. The effect of topical therapy with exemplary preparation containing lipopeptide conjugates in an effective amount on wound healing is shown in Figures (Fig. 8, Fig. 9, Fig. 10, Fig. 11). Influence of polimer matrix used to prepare wound-dressing on the overal release, including the rate of lipopeptides release from polimer matrix was determined.

To better understand how topical therapy affected wound healing processes, the study at the cellular level/scale was performed. *In vitro* studies showed that topical therapy with pharmaceutical compositions containing lipopeptide conjugates or their pharmaceutically acceptable salts could enhance cellular viability (activate skin cells processes). Important process in the proliferative phase is re-epithelization, which involves keratinocyte proliferation and migration from the wound edges to the center of the wound. The results showed that pharmaceutical compositions cotaining lipopeptide conjugates or their pharmaceutically acceptable salts are able to induce/stimulate proliferation process, initiate and modulate migration rate of skin cells. These data suggest that a dominating process mediated by keratinocytes is proliferation.

XTT cell viability assay proved the stimulation of cell growth by pharmaceutical compositions containing lipoeptide conjugates or their pharmaceutically acceptable salts. These results indicate additional roles of topical therapy in proliferative and remodelling phase of wound healing. The effect of pharmaceutical compositions according to the invention on cell viability of fibroblasts may have a role in promoting fibroblast-induced collagen depositon and other ECM components during the proliferative and tissue remodelling phase. Fibroblast migration rate in response to lipopeptide conjugate therapy was examined via *in vitro* wound healing assay and visualized microscopically. The wound area reduced as cell migration progresses during 24 hours, compared to the control (Fig. 4). Figure 7 shows microscopic images of wound closure (human primary fibroblast cells) induced with pharmaceutical composition comprising lipopeptide conjugate.

Histology analysis revealed properly restoration tissue in experimental group treated with pharmaceutical compositions containing lipopeptide conjugates. Microscopic evaluation on Day 10 proved the participation of lipopeptide conjugates in restoration of normal skin architecture during wound healing (the formation of a new epithelial layer and granulation tissue). From representatives images of Masson's trichrome-stained section of mouse skin treated with lipopeptide conjugate the thickening of the epidermal layer was visible. This fact indicates and intesified activation of the keratinocyte proliferation process. thickness is a result of wounding and rise in the number of keratinocytes (cell proliferation). The evidence of collagen and elastin fibres proved the increased collagen production by fibroblast cells.

The application of hydrogel wound-dressings according to the invention in mice wounds provide delivery system of lipopeptide conjugate release. The wound healing effect of preparations, containing lipopeptide conjugates in mice was shown in Figure 12. The results presented representative example of accelerated wound closure in mice treated with preparation compared to the control. Wounds from mice were nearly completely healed within 10 days, as was seen with FA₂ treatment, having only 7.44% of the wound area remaining open. The effect of applied hydrogel wound dressing FA₁-FA₃ on full-layered wound in diabetic mice (*dbldb*) was shown in Figures (Fig. 14, Fig. 15, Fig. 16). A progressive reepitalization process was observed from Day 3 after treatment with preparations. Mice showed a decrease in wound size on day 10. The percentage of the open wound size and re-epithelialized wound areas in experimental group treated with wound hydrogel dressings FA₁, FA₂, FA₃ on day 10 were calculated as 17.18% and 41.26% (FA₁); 13.80% and 39.12% (FA₂); 13.36% and 41,27% (FA₃). The rate of healing, or area healed on day 10 in the following experimental groups was calculated as 41,56% (FA₁), 47,08% (FA₂), 45,37% (FA₃). In this type of healing, a full-thickness wound allowed to close on day 18.

The regenerative potential in diabetes-induced delayed wound healing model was noticed on day 7 under treatment with a hydrogel wound dressing (FA₄).

Diagram (Fig. 17) presents a comparison of the percentage of the open wound size and area healed in non-diabetic and diabetic mice during the time intervals of experiments.

The area healed in diabetic mice on day 7 (in the control group) was calculated as 11.94% (of initial wound), however in non-diabetic mice was calculated as 47.99%.

The rate of healing increased as the area healed in diabetic mice on Day 10 was calculated as 25.29%. Application of the hydrogel wound dressing (FA₁-FA₃) according to the invention enhance the healing process of diabetic wounds. Macroscopic analysis of wound closure in mice on day 14 under treatment with hydrogel wound dressings proved the completness of healing. Representative photographs of wounds treated with FA₂ compared to the control FA₄ were shown in Fig. 17

The results proved biological activity and safety profile of lipopeptide conjugates in *in vitro, in vivo* studies. This therapy reported no serious side effects, including system adverse effect or harmful, irritiating effect on the skin of the mice. No behavioral changes was also noticed.

### Statistical data analysis.

The data are expressed as the mean±SEM. A significant differences in mean values between groups were analyzed using Student's t-test or ANOVA and non-parametric Mann-Whitney U test.

## Claims

1. A novel hydroxyproline-rich lipopeptide conjugate of general formula:
*N*^{α}-acyl-(AK₅-AK₄-AK₃-AK₂-AK₁)ₙ-NH₂ (I)
or its pharmaceutically acceptable salt, wherein:
AK₁,AK₂,AK₃,AK₄,AK₅ denote amino acid residues or their lack;
**AK₁** is the lysine residue of the L or D configuration.
**AK₂** is an amino acid residue selected from the group consisting of non-polar neutral amino acids:
Phe, Ala, Gly, Ile, Leu, Met, Pro, Val, Trp or its lack;
**AK₃** is an amino acid residue selected from Hyp, Phe, Ala, Gly, Ile, Leu, Met, Pro, Val, Trp;
**AK₄** is an amino acid residue selected from Pro, Hyp or its lack
**AK₅** is the Gly residue;
***N*^{α}-acyl** group has the formula R₁-C(=O), wherein R₁ is a saturated carbon chain of length from C₇ to C₁₃;
**n** denotes a number of repeat units from 1 to 2.

2. A lipopeptide conjugate according to claim 1, **characterized in that** the amide form of the conjugate or its pharmaceutically acceptable salt , the salt being selected from trifluoroacetate, acetate, hydrochloride, hydrobromide, hydroiodide, hydrofluoride, has the following sequences:
**Table 1.**
| ***Lp.*** | ***N*^{α}-acyl = R₁-C(=O)** | **AK₅** | **AK₄** | **AK₃** | **AK₂** | **AK₁** | **AK₄'** | **AK₃'** | **AK₂'** | **AK₁'** | **n** |
|---|---|---|---|---|---|---|---|---|---|---|---|
| 1 | C₁₁ | Gly | - | Pro | - | L-Lys | - | - | - | - | 1 |
| 2 | C₁₁ | Gly | Hyp | Hyp | - | L-Lys | - | - | - | - | 1 |
| 3 | C₁₁ | Gly | Pro | Hyp | - | L-Lys | - | - | - | - | 1 |
| 4 | C₁₁ | Gly | Hyp | Ala | - | L-Lys | - | - | - | - | 1 |
| 5 | C₁₁ | Gly | Hyp | Hyp | - | D-Lys | - | - | - | - | 1 |
| 6 | C₁₁ | Gly | Hyp | Pro | - | L-Lys | - | - | - | - | 1 |
| 7 | C₇ | Gly | Hyp | Hyp | - | L-Lys | - | - | - | - | 1 |
| 8 | C₁₁ | Gly | Pro | Hyp | Gly | L-Lys | - | - | - | - | 1 |
| 9 | C₁₁ | Gly | Pro | Hyp | Met | L-Lys | - | - | - | - | 1 |
| 10 | C₁₁ | Gly | Pro | Hyp | Trp | L-Lys | - | - | - | - | 1 |
| 11 | C₁₁ | Gly | Hyp | Gly | Pro | L-Lys | - | - | - | - | 1 |
| 12 | C₁₁ | Gly | Pro | Hyp | Ile | L-Lys | - | - | - | - | 1 |
| 13 | C₁₁ | Gly | Pro | Hyp | Phe | L-Lys | - | - | - | - | 1 |
| 14 | C₁₁ | Gly | Pro | Hyp | Ala | L-Lys | - | - | - | - | 1 |
| 15 | C₁₁ | Gly | Pro | Hyp | Leu | L-Lys | - | - | - | - | 1 |
| 16 | C₁₁ | Gly | Hyp | Hyp | Val | L-Lys | - | - | - | - | 1 |
| 17 | C₁₁ | Gly | Hyp | Hyp | Pro | L-Lys | - | - | - | - | 1 |
| 18 | C₁₁ | Gly | Hyp | Leu | Pro | L-Lys | - | - | - | - | 1 |
| 19 | C₁₁ | Gly | Hyp | Met | Pro | L-Lys | - | - | - | - | 1 |
| 20 | C₁₁ | Gly | Hyp | Ala | Pro | L-Lys | - | - | - | - | 1 |
| 21 | C₁₁ | Gly | Hyp | Hyp | Gly | L-Lys | - | - | - | - | 1 |
| 22 | C₁₁ | Gly | Hyp | Hyp | Met | L-Lys | - | - | - | - | 1 |
| 23 | C₁₁ | Gly | Hyp | Hyp | Trp | L-Lys | - | - | - | - | 1 |
| 24 | C₁₁ | Gly | - | Hyp | Gly | L-Lys | - | - | - | - | 1 |
| 25 | C₁₁ | Gly | Hyp | Hyp | - | L-Lys | Pro | Hyp | - | L-Lys | 2 |
| 26 | C₁₁ | Gly | Hyp | Hyp | - | L-Lys | Hyp | Hyp | - | L-Lys | 2 |
| 27 | C₁₁ | Gly | - | Pro | - | L-Lys | Pro | Hyp | - | L-Lys | 2 |
| 28 | C₁₁ | Gly | - | Pro | - | L-Lys | Hyp | Hyp | - | L-Lys | 2 |
| 29 | C₁₁ | Gly | Pro | Hyp | - | L-Lys | Hyp | Hyp | - | L-Lys | 2 |
| 30 | C₁₁ | Gly | Pro | Hyp | - | L-Lys | Hyp | Hyp | - | D-Lys | 2 |
| 31 | C₇ | Gly | Pro | Hyp | - | L-Lys | Hyp | Hyp | - | L-Lys | 2 |
| 32 | C₉ | Gly | Pro | Hyp | - | L-Lys | Hyp | Hyp | - | L-Lys | 2 |
| 33 | C₁₃ | Gly | Pro | Hyp | - | L-Lys | Hyp | Hyp | - | L-Lys | 2 |
| 34 | C₁₁ | Gly | Pro | Hyp | - | L-Lys | Pro | Hyp | Gly | L-Lys | 2 |
| 35 | C₁₁ | Gly | Pro | Hyp | - | L-Lys | Hyp | Gly | - | L-Lys | 2 |
| 36 | C₁₁ | Gly | Pro | Hyp | - | L-Lys | - | Hyp | Gly | L-Lys | 2 |

3. The method of obtaining, on a polymeric carrier, according to the Fmoc strategy a lipopeptide conjugate of formula I defined in claims 1 and 2, wherein n = 1, proceeds in the following steps: the coupling reaction of an active ester of the protected amino acid residue with a suitably activated polymeric carrier, attachment of the protected amino acid residues to obtain the defined amino acid sequences in two steps: Fmoc deprotection using a 25% piperidine solution in DMF and coupling reaction using a carbodiimide derivative in a DCM/DMF solvent mixture, and the attachment of a long-chain fatty acid to an *N*^{α}-terminus of the peptidyl carrier is performed using a uronic-type salt and an anti-racemic reagent under alkaline conditions and, finally, the cleavage of peptide conjugated with fatty acid from the carrier by treatment of the carrier with TFA at different concentrations of 1-95%, **characterized in that**
a) the coupling reaction of an active ester of Fmoc-L-Lys(Boc)-OH or Fmoc-D-Lys(Boc)-OH with an activated aminomethylpolystyrene via a 4-(2',4'-dimethoxyphenyl-Fmoc-aminomethyl)-phenoxyacetamide linker;
b) attachment of the Fmoc-protected amino acid residues to obtain the sequences defined in claim 2 is carried out under anhydrous conditions at 25±2°C for 3÷5 hours with DIPCI and HOBt in a molar ratio 1:1:1 using 2.5÷5.0-fold excess of reactants in relation to the carrier loading in a NMP-DMF-DCM solvent mixture (1:1:1, *v*/*v*/*v*);
(c) attachment of a long chain fatty acid (C₈÷C₁₄) to an *N*^{α}-terminus of the peptidyl carrier is performed under anhydrous conditions at 25±2°C for 3÷5 h with TBTU, HOBt and DIPEA at a molar ratio of 1:1:1:2 using a 2.5-fold excess of reactants in relation to the carrier loading in DMF with the addition of t-octylphenoxypolyethoxyethanol non-ionic surfactant (1%) (Cmc=267, HLB=14,4);
(d) cleavage of peptide conjugated with fatty acid from the polymeric carrier is carried out under inert gas, preferably nitrogen, in the dark for 2÷3 hours in a mixture of concentrated TFA, scavenger and/or antioxidant;
(e) purification of the cleaved lipopeptide conjugate is carried out by reversed-phase solid phase extraction.

4. The method of obtaining on a polymeric carrier according to the Fmoc strategy a lipopeptide conjugate of formula I defined in claims 1 and 2, wherein n = 2, proceeds in the following steps: the (poly)condensation reaction of N^{α}-acylpeptide with an α-amino group of the peptide chain fragment loaded the carrier, and the attachment of a long-chain fatty acid to an *N*^{α}-terminus of peptidyl carrier is performed using a uronic-type salt and an anti-racemic reagent under alkaline conditions and, finally, the cleavage of peptide conjugated with fatty acid from the carrier by treatment of the carrier with a TFA at concentrations in the range from 1% to 95%, **characterized in that** in parallel two peptide chains A and B are obtained on a 2-chlorotrityl carrier (fragment A) and aminomethylpolystyrene carrier (fragment B), respectively in the following reactions:
(a) coupling reaction of the active ester of Fmoc-L-Lys(Boc)-OH or Fmoc-D-Lys(Boc)-OH with an activated polymeric carrier which is polystyrene having as linker a 2-chlorotrityl ester (fragment A) and aminomethylpolystyrene having as linker a 4-(2',4'-dimethoxyphenyl-Fmoc-aminomethyl)-phenoxyacetamide (fragment B) respectively;
(b) attachment of the Fmoc protected amino acid residues to obtain the defined peptide fragment A and peptide fragment B is carried out under anhydrous conditions, at 25±2°C for 3÷5 hours with DIPCI and HOBt at a molar ratio of 1:1:1 using 2.5÷5.0 fold excess of reactants in relation to the carrier loading in a NMP-DMF-DCM solvent mixture (1:1:1, *v*/*v*/*v*);
(c) attachment of a long chain fatty acid (C₈÷C₁₄) to an *N*^{α}-terminus of the peptidyl carrier (fragment A loaded carrier) is performed under anhydrous conditions at 25±2°C for 3÷5 h with TBTU, HOBt in the presence of DIPEA at a molar ratio of 1:1:1:2 using 2.5-fold excess of reactants in relation to the carrier loading, in DMF with the addition of t-octylphenoxypolyethoxyethanol non-ionic surfactant (1%) (Cmc=267, HLB=14,4);
(d) cleavage of the lipopeptide chains (lipo-fragment A) from the carrier by treatment of the polymeric carrier with DCM-TFA (95:5, v/v) or DCM-TFA-iPr₃SiH (90:5:5, v/v/v) in an inert gas, preferably nitrogen, in the dark for 30 minutes, followed by neutralisation of the cleaved lipopeptide chains and purification,
(e) polycondensation reaction of peptide fragments: purified lipo-fragment A and fragment B loaded aminomethylpolystyrene carrier is performed under anhydrous conditions at 25±2°C for 2.5÷5.0 hours using TBTU, HOBt in the presence of DIPEA in a molar ratio 1:2:2:4, using 2.5÷8.0 fold excess of reactants in relation to the carrier loading, in DMF;
(f) cleavage of the lipopeptide conjugate defined in claim 2 from the carrier is carried out under an inert gas, preferably nitrogen, in the dark, for a period of 2÷3 hours in the presence of a mixture of concentrated TFA, scavenger and/or antioxidant;
(g) purification of the cleaved lipopeptide conjugate is carried out by reversed-phase solid phase extraction.

5. The method according to claim 3 or claim 4, **characterized in that** loading of the Fmoc-L-Lys(Boc)-OH or Fmoc-D-Lys(Boc)-OH on the 2-chlorotrityl carrier and aminomethylpolystyrene carrier is in the ranges of (0.655÷1.245)±0.040mmol/g, and (0.345÷0.790) ±0.050mmol/g, respectively.

6. The method according to claim 3 or claim 4, **characterized in that** L-hydroxyproline-rich lipopeptide conjugates defined in claims 1 and 2 are cleaved from an aminomethylpolystyrene carrier using a mixture of concentrated TFA and scavenger: TFA-PhOH-iPr₃SiH-H₂O (88:5.8:2:4.2, *v*/*v*/*v*/*v*) for a period of 2÷3h, at 25±2°C or a mixture of concentrated TFA, scavenger and antioxidant: TFA-PhOH-iPr₃SiH-H₂O with the addition of NH₄I (86.5:5.8:4.2:1.5, *v*/*v*/*v*/*v*) for a period of 3÷4h at 0°C.

7. The method according to claims from 3 to 6, **characterized in that** L-hydroxyproline-rich lipopeptide conjugates as defined in claims 1 and 2 are obtained using Fmoc-L-4-hydroxyproline.

8. The method according to claims from 3 to 7, **characterized in that** the coupling reaction between the free α-amino group of the amino acid residue in the sequence of L-4-hydroxyproline-containing peptidyl carrier and the carboxyl group of the successively coupled Fmoc-protected amino acid residue to build hydroxyproline-rich lipopeptide conjugates as defined in claims 1 and 2, wherein Fmoc-protected amino acid, DIPCI and HOBt are used in a molar ratio of 2:2:2, and the reaction is carried out in a NMP-DMF-DCM solvent mixture (1:1:1, *v*/*v*/*v*) at 18±2°C, for a period of 3.5±0.5 hours.

9. The method according to claim 3 or 4 **characterized in that** the lipopeptide conjugates cleaved from the polymeric carrier are purified by reversed-phase solid phase extraction using columns packed with the octadecyl group bonded type silica gel, with gradient elution at concentrations in the range from 0 to 30% of acetonitrile in water with addition of 0.1% trifluoroacetic acid and isocratic elution.

10. A pharmaceutical composition, **characterized in that** it comprises at least one lipopeptide conjugate or its pharmaceutically acceptable salt as defined in claims 1 and 2 in the amount varies from 0.0001% to 10% and at least one pharmaceutically acceptable solvent.

11. A preparation for stimulating proliferation and migration of fibroblasts and keratinocytes, **characterized in that** it contains the pharmaceutical composition defined in claim 10 in the amount varies from 0.01% to 10% by weight, of the total weight of a wound dressing.

12. The use of a pharmaceutical composition as defined in claim 10 comprising a lipopeptide conjugate or its pharmaceutically acceptable salt as defined in claims 1 and 2, or a preparation as defined in claim 11, as a basic component for the manufacture of a wound dressing in the form of a solution, a suspension, an aerosol, an emulsion, a cream, an ointment, a three-dimensional scaffold, a hydrogel in a tube.

13. The use of a pharmaceutical composition as defined in claim 10 comprising a lipopeptide conjugate or its pharmaceutically acceptable salt as defined in claims 1 and 2 or a preparation as defined in claim 11 or a wound dressing as defined in claim 12 for the treatment of chronic wounds, advantageously burn wounds, venous leg ulcers, diabetic foot, as well as those which are a complication of existing cardiovascular disease or diabetes mellitus.
